# EUROPEAN PATENT APPLICATION

(11) **EP 3 766 901 A1**
(43) Date of publication of application: **20.01.2021**
(21) Application number: 19767333.8
(22) Date of filing: 13.03.2019
(51) Int. Cl.: C07K 16/28, C12N 15/13, A61K 39/44, A61P 35/00

(54) **ANTIBODY TARGETING CTLA-4 , PREPARATION METHOD THEREFOR AND USE THEREOF**

(30) Priority: 14.03.2018 CN 201810207977
(71) Applicant: Shanghai Pharmaexplorer Co., Ltd., Shanghai 201210 (CN); Pharmaexplorer Limited, Road Town, Tortola (VG)
(72) Inventor: XU, Lina, Shanghai 201210 (CN); LIU, Lile, Shanghai 201210 (CN); YANG, Tatchi Teddy, Shanghai 201210 (CN); WEI, Yuxing, Shanghai 201210 (CN); SHAO, Xiaohui, Shanghai 201210 (CN); WANG, Qian, Shanghai 201210 (CN); ZHANG, Jie, Shanghai 201210 (CN); WANG, Meiling, Shanghai 201210 (CN); ZHANG, Yu, Shanghai 201210 (CN); DUAN, Qing, Shanghai 201210 (CN); SONG, Ningning, Shanghai 201210 (CN)
(74) Representative: Mewburn Ellis LLP
(86) International application number: PCT/CN2019/078032
(87) International publication number: WO 2019/174603

(57) **Abstract**

Disclosed by the present invention are an antibody targeting CTLA-4, a preparation method therefor and a use thereof. In particular, disclosed by the present invention is a novel fully human monoclonal antibody that targets CTLA-4. Further disclosed by the present invention is a method for preparing the monoclonal antibody. The monoclonal antibody of the present invention is capable of binding a CTLA-4 antigen with high specificity, has a high affinity, remarkable anti-tumor activity, and so on.

## Description

### Technical field

The present invention relates to the field of biomedicine, more particularly relates to a CTLA-4 antibody and a preparation method and application thereof.

### Background

The application of monoclonal antibodies is one of the most successful and transformative treatments in cancer treatment in the past 20 years. Compared with traditional chemical drugs, antibody drugs have higher specificity and lower toxicity. Although monoclonal antibody drugs have achieved continuous success, they still face many challenges.

The biggest defect of a murine monoclonal antibody is the HAMA (human anti-mouse antibody) response it induces. Therefore, murine monoclonal antibodies have great limitations in the diagnosis and treatment of tumors, organ transplantation and other diseases; chimeric antibodies still retain 30% of the murine sequence, which can cause different degrees of HAMA response. Clinically, different chimeric antibodies have different degrees of immunogenicity; humanized antibodies are also called transplantation antibodies. Simple CDR transplantation often leads to a decrease in antigen-antibody affinity. Because it still has at least 10% heterologous protein, its clinical application is still limited to varying degrees. Therefore, it is necessary to further develop a more perfect therapeutic antibody, a fully human antibody.

In 1994, two American companies Abgenix and Genpham reported the use of transgenic mice to prepare fully human antibodies, thus solving the problem of human antibody preparation research that the human body cannot be immunized at will. Since then, the technology for preparing fully human antibodies has continued to develop and mature, and the obtained human monoclonal antibodies have strong anti-tumor activity.

Cancer immunotherapy is the latest breakthrough in cancer treatment, which uses the patient's own immune system to attack tumor cells.

Immune checkpoints refer to some inhibitory signal pathways in the immune system, which prevent tissue damage by regulating the persistence and intensity of immune responses in peripheral tissues, and participate in maintaining tolerance to self-antigens. The use of inhibitory signaling pathways of immune checkpoints to inhibit T cell activity is an important mechanism for tumors to escape immune killing. Blocking immune checkpoints is one of many effective strategies to activate anti-tumor immunity.

Inhibitors of immune checkpoint proteins have the potential to treat various tumor types (such as metastatic melanoma, lung cancer, breast cancer, renal cell carcinoma, etc.). Recent research on cancer immunotherapy has shown promising results, especially for metastatic cancer cases. In addition, cancer immunotherapy has great potential in the treatment of blood cancers, including Hodgkin's lymphoma, multiple myeloma, myelodysplastic syndrome, and non-Hodgkin's lymphoma. The side effects caused by immune checkpoint inhibitors are negligible, reversible and controllable, and effective immune checkpoint inhibitors can significantly improve the overall survival of cancer patients. Immune checkpoint inhibitors can be used in combination with targeted therapy or conventional radiotherapy and chemotherapy, and this combination therapy can effectively treat many types of cancer.

CTLA-4 (Cytotoxic T Lymphocyte-Associated Protein 4 or CD152) is a type I transmembrane protein as one of the main immune checkpoint molecules, which contains an extracellular Ig-V-like domain, an Ig-C-like domain, a transmembrane domain and an intracellular C-terminal domain. CTLA-4 is mainly expressed constitutively in regulatory T cells and expressed inducibly on the surface of activated T cells, and interacts with ligands B7.1 (CD80) and B7.2 (CD82) to induce inhibitory signals, which leads to T cell activation, inhibition of proliferation and reduction of cytokine production. CTLA-4 is constitutively highly expressed on the surface of Treg cells and participates in the down-regulation of the activity of other effector T cells by Treg.

CTLA-4 is crucial in the regulation of activity of T cells in the early stage of T cell activation, and thus becomes the main regulatory mechanism of immune tolerance in the peripheral lymphatic system. CTLA-4 competes with CD28 for ligands and inhibits the TCR signaling pathway by itself, thus regulating the intensity of T cell response to antigens, so that the body shows tolerance to self-antigens or foreign weak antigen recognition.

Studies believe that blocking the CTLA-4/B7 ligand interaction can improve the body's ability to recognize tumor antigens and stimulate the proliferation of antigen-specific T cells, thereby activating the immune system and enhancing the anti-tumor immune response. It has been proved in several mice tumor models of the same strain that CTLA-4 blockade promotes anti-tumor activity. Therefore, drugs blocking CTLA-4/B7.1 and B7.2 pathways can provide new treatments for various cancers and other immune diseases.

Currently, the existing CTLA-4 antibodies in the field still have many shortcomings, such as small therapeutic windows, limited indications, and high treatment costs. Therefore, the development of CTLA-4 blocking antibodies is an urgent need for the treatment of various cancers.

### Summary of the invention

In order to overcome the current lack of fully human CTLA-4 antibodies and the shortcomings of existing CTLA-4 antibodies in terms of activity and safety, the present invention provides a CTLA-4 antibody with high affinity and strong specificity and a preparation method thereof.

In a first aspect of the present invention, it provides a heavy chain variable region of an antibody, wherein the heavy chain variable region comprises the following three complementary determining regions or CDRs:
CDR1 shown in SEQ ID NO: 8n+2,
CDR2 shown in SEQ ID NO: 8n+3, and
CDR3 shown in SEQ ID NO: 8n+4;
wherein, each n is independently 0, 1, 2, 3 or 4;
wherein, any one of the above amino acid sequences also includes a derivative sequence that is optionally with at least one amino acid added, deleted, modified, and/or substituted, and is capable of retaining the binding affinity to CTLA-4.

In another preferred embodiment, the heavy chain variable region has the amino acid sequence shown in SEQ ID NO: 8n+1, wherein n is 0, 1, 2, 3 or 4.

In a second aspect of the present invention, it provides a heavy chain of an antibody, wherein the heavy chain comprises the heavy chain variable region according to claim 1.

In a third aspect of the present invention, it provides a light chain variable region of an antibody, wherein the light chain variable region comprises the following three complementary determining regions or CDRs:
CDR1' shown in SEQ ID NO: 8n+6,
CDR2' shown in SEQ ID NO: 8n+7, and
CDR3' shown in SEQ ID NO: 8n+8;
wherein, each n is independently 0, 1, 2, 3 or 4;
wherein, any one of the above amino acid sequences also includes a derivative sequence that is optionally with at least one amino acid added, deleted, modified, and/or substituted, and is capable of retaining the binding affinity to CTLA-4.

In another preferred embodiment, the light chain variable region has the amino acid sequence shown in SEQ ID NO: 8n+5, wherein n is 0, 1, 2, 3 or 4.

In a fourth aspect of the present invention, it provides a light chain of an antibody, wherein the light chain comprises the light chain variable region according to claim 3.

In a fifth aspect of the present invention, it provides an antibody, wherein the antibody comprises:
(1) the heavy chain variable region according to the first aspect of the present invention; and/or
(2) the light chain variable region according to the third aspect of the present invention;
or the antibody comprises: the heavy chain according to the second aspect of the present invention; and/or the light chain according to the fourth aspect of the present invention,
wherein, any one of the above amino acid sequences also includes a derivative sequence that is optionally with at least one amino acid added, deleted, modified, and/or substituted, and is capable of retaining the binding affinity to CTLA-4.

In another preferred embodiment, the amino acid sequence of any of the above-mentioned CDRs includes a derivative CDR sequence with 1, 2 or 3 amino acids added, deleted, modified and/or substituted, and the derivative antibody comprising the VH and VL containing the derivative CDR sequence can retain the binding affinity to CTLA-4.

In another preferred embodiment, the ratio (F1/F0) of the binding affinity F1 between the derivatized antibody and CTLA-4 to the binding affinity F0 between the corresponding non-derivatized antibody and CTLA-4 is 0.5-2, preferably 0.7-1.5, and more preferably 0.8-1.2.

In another preferred embodiment, the number of added, deleted, modified and/or substituted amino acids is 1-5 (such as 1-3, preferably 1-2, more preferably 1).

In another preferred embodiment, the derivative sequence with at least one amino acid added, deleted, modified, and/or substituted, which can retain the binding affinity to CTLA-4, is an amino acid sequence having a homology of at least 96%.

In another preferred embodiment, the antibody further comprises a heavy chain constant region and/or a light chain constant region.

In another preferred embodiment, the heavy chain constant region is of human, and/or the light chain constant region is of human.

In another preferred embodiment, the antibody is selected from: chimeric antibodies, humanized antibodies, fully human antibodies, and a combination thereof.

In another preferred embodiment, the ratio (Z1/Z0) of the immunogenicity Z1 of the fully human antibody in human to the immunogenicity Z0 of the non-fully human antibody (such as murine-derived antibody) in human is 0-0.5, preferably 0-0.2, more preferably 0-0.05 (e.g. 0.001-0.05).

In another preferred embodiment, the antibody is a partially or fully humanized or fully human monoclonal antibody.

In another preferred embodiment, the antibody is a double-chain antibody or a single-chain antibody.

In another preferred embodiment, the antibody is a full-length antibody protein or an antigen-binding fragment.

In another preferred embodiment, the antibody is a bispecific antibody or a multispecific antibody.

In another preferred embodiment, the antibody has one or more properties selected from the following group:
(a) inhibiting tumor cell migration or metastasis;
(b) inhibiting tumor growth.

In another preferred embodiment, the antibody comprises the heavy chain variable region according to the first aspect of the present invention and the light chain variable region according to the third aspect of the present invention;
wherein, the heavy chain variable region comprises the following three complementary determining regions or CDRs:
CDR1 shown in SEQ ID NO: 2,
CDR2 shown in SEQ ID NO: 3, and
CDR3 shown in SEQ ID NO: 4;
wherein, the light chain variable region comprises the following three complementary determining regions or CDRs:
CDR1' shown in SEQ ID NO: 6,
CDR2' shown in SEQ ID NO: 7, and
CDR3' shown in SEQ ID NO: 8;
wherein, any one of the above amino acid sequences also includes a derivative sequence that is optionally with at least one amino acid added, deleted, modified, and/or substituted, and is capable of retaining the binding affinity to CTLA-4.

In another preferred embodiment, the heavy chain variable region of the antibody comprises the amino acid sequence shown in SEQ ID NO: 8n+1; and/or the light chain variable region of the antibody comprises the amino acid sequence shown in SEQ ID NO: 8n+5, wherein each n is independently 0, 1, 2, 3 or 4.

In another preferred embodiment, the heavy chain variable region of the antibody has the amino acid sequence shown in SEQ ID NO: 1.

In another preferred embodiment, the light chain variable region of the antibody has the amino acid sequence shown in SEQ ID NO: 5.

In another preferred embodiment, the heavy chain variable region of the antibody has the amino acid sequence shown in SEQ ID NO: 1, and the light chain variable region of the antibody has the amino acid sequence shown in SEQ ID NO: 5.

In another preferred embodiment, the heavy chain variable region of the antibody has the amino acid sequence shown in SEQ ID NO: 9, and the light chain variable region of the antibody has the amino acid sequence shown in SEQ ID NO: 13.

In another preferred embodiment, the heavy chain variable region of the antibody has the amino acid sequence shown in SEQ ID NO: 17, and the light chain variable region of the antibody has the amino acid sequence shown in SEQ ID NO: 21.

In another preferred embodiment, the heavy chain variable region of the antibody has the amino acid sequence shown in SEQ ID NO: 25, and the light chain variable region of the antibody has the amino acid sequence shown in SEQ ID NO: 29.

In another preferred embodiment, the heavy chain variable region of the antibody has the amino acid sequence shown in SEQ ID NO: 33, and the light chain variable region of the antibody has the amino acid sequence shown in SEQ ID NO: 37.

In another preferred embodiment, the antibody is selected from the following group consisting of: 97A8D1, 92C8B6, 31C12F3, 40C4C6 and 97B8E1.

The amino acid sequence of the heavy chain variable region has a sequence homology of at least 80%, 85%, 90%, 91%, 92%, 93%, 94%, 95%, 96%, 97%, 98% or 99% with the amino acid sequence shown in SEQ ID NO: 1, SEQ ID NO: 9, SEQ ID NO: 17, SEQ ID NO: 25, or SEQ ID NO: 33 in the sequence listing.

The amino acid sequence of the light chain variable region has a sequence homology of at least 80%, 85%, 90%, 91%, 92%, 93%, 94%, 95%, 96%, 97%, 98% or 99% with the amino acid sequence shown in SEQ ID NO: 5, SEQ ID NO: 13, SEQ ID NO: 21, SEQ ID NO: 29, or SEQ ID NO: 37 in the sequence listing.

In another preferred embodiment, the antibody is a defucosylated antibody.

In another preferred embodiment, the antibody is defucosylated antibody 97A8D1.

In a sixth aspect of the present invention, it provides a recombinant protein, wherein the recombinant protein comprises:
(i) the heavy chain variable region according to the first aspect of the present invention, the heavy chain according to the second aspect of the present invention, the light chain variable region according to the third aspect of the present invention, the light chain according to the fourth aspect of the present invention, or the antibody according to any one of the fifth aspect of the present invention; and
(ii) an optional tag sequence to assist expression and/or purification.

In another preferred embodiment, the tag sequence includes a 6His tag.

In another preferred embodiment, the recombinant protein (or polypeptide) includes a fusion protein.

In another preferred embodiment, the recombinant protein is a monomer, dimer, or multimer.

In another preferred embodiment, the recombinant protein comprises:
(i) the antibody according to the fifth aspect of the present invention, wherein the heavy chain variable region of the antibody has the amino acid sequence shown in SEQ ID NO: 1, and the light chain variable region of the antibody has the amino acid sequence shown in SEQ ID NO: 5, and
(ii) an optional tag sequence to assist expression and/or purification.

In another preferred embodiment, the recombinant protein comprises:
(i) a defucosylated antibody (such as defucosylated antibody 97A8D1);
(ii) an optional tag sequence to assist expression and/or purification.

In a seventh aspect of the present invention, it provides a polynucleotide, which encodes a polypeptide selected from the group consisting of:
(1) the heavy chain variable region according to the first aspect of the present invention, the heavy chain according to the second aspect of the present invention, the light chain variable region according to the third aspect of the present invention, the light chain according to the fourth aspect of the present invention, or the antibody according to any one of the fifth aspect of the present invention; and
(2) the recombinant protein according to the sixth aspect of the present invention.

In another preferred embodiment, the polynucleotide encoding the heavy chain variable region is shown in SEQ ID NO: 41, 43, 45, 47 or 49; and/or,
the polynucleotide encoding the light chain variable region is shown in 42, 44, 46, 48 or 50.

In another preferred embodiment, the polynucleotide encoding the heavy chain variable region sequence is shown in SEQ ID NO: 41; and the polynucleotide encoding the light chain variable region sequence is shown in 42.

In another preferred embodiment, the polynucleotide encoding the heavy chain variable region is shown in SEQ ID NO: 43; and the polynucleotide encoding the light chain variable region is shown in 44.

In another preferred embodiment, the polynucleotide encoding the heavy chain variable region is shown in SEQ ID NO: 45; and the polynucleotide encoding the light chain variable region is shown in 46.

In another preferred embodiment, the polynucleotide encoding the heavy chain variable region is shown in SEQ ID NO: 47; and the polynucleotide encoding the light chain variable region is shown in 48.

In another preferred embodiment, the polynucleotide encoding the heavy chain variable region is shown in SEQ ID NO: 49; and the polynucleotide encoding the light chain variable region is shown in 50.

In an eighth aspect of the present invention, it provides a vector, which contains the polynucleotide according to any one of the seventh aspect of the present invention.

In another preferred embodiment, the vector includes: bacterial plasmid, phage, yeast plasmid, plant cell virus, mammalian cell virus such as adenovirus, retrovirus, or other vectors.

In a ninth aspect of the present invention, it provides a genetically engineered host cell, wherein the host cell contains the vector according to the eighth aspect of the present invention or the genome thereof is integrated with the polynucleotide according to any one of the seventh aspect of the present invention..

In a tenth aspect of the present invention, it provides a pharmaceutical composition, wherein the pharmaceutical composition comprises:
(i) an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, and the antibody according to any one of the fifth aspect of the present invention, the recombinant protein according to the sixth aspect of the present invention, and combinations thereof; and
(ii) a pharmaceutically acceptable carrier.

In another preferred embodiment, the pharmaceutical composition is a liquid preparation.

In another preferred embodiment, the pharmaceutical composition is an injection.

In another preferred embodiment, the pharmaceutical composition comprises 0.01-99.99% of the antibody according to any one of the fifth aspect of the present invention, the recombinant protein according to the sixth aspect of the present invention, or a combination thereof, and 0.01-99.99% of the pharmaceutically acceptable carrier, wherein the percentage is the mass percentage of the pharmaceutical composition.

In an eleventh aspect of the present invention, it provides use of an active ingredient, wherein the active ingredient is selected from the group consisting of: the heavy chain variable region according to the first aspect of the present invention, the heavy chain according to the second aspect of the present invention, the light chain variable region according to the third aspect of the present invention, the light chain according to the fourth aspect of the present invention, and the antibody according to any one of the fifth aspect of the present invention, the recombinant protein according to the sixth aspect of the present invention, and combinations thereof, wherein the active ingredient is used for preparation of a medicine for preventing and/or treating CTLA-4 related cancers and/or viral infection related diseases.

In another preferred embodiment, the CTLA-4 related disease is selected from the group consisting of melanoma, mesothelioma, non-small cell lung cancer, breast cancer, liver cancer, synovial sarcoma, metastatic colon cancer, kidney cancer, bladder cancer, prostate cancer, ovarian cancer, chronic hepatitis C virus infection, advanced solid cancer, malignant tumors of digestive organs, metastatic non-small cell lung cancer, prostate tumors, endometrial carcinoma, endometrial carcinosarcoma, recurrent melanoma, head and neck squamous cell carcinoma, sarcoma, Merkel cell carcinoma, skin T-cell lymphoma, fallopian tube cancer, peritoneal tumor, muscle invasive bladder cancer, extensive stage small cell lung cancer, adult acute myeloid leukemia, atypical chronic myelogenous leukemia, previously treated myelodysplastic syndrome, epithelial ovarian cell carcinoma, urinary system malignancies, adult grade III lymphoma-like granuloma, B-cell chronic lymphocytic leukemia, skin B-cell non-Hodgkin's lymphoma, intraocular lymphoma, choriocarcinoma of testis, neuroblastoma, esophageal cancer, etc.

In a twelfth aspect of the present invention, it provides a composition for detecting CTLA-4 protein in a sample *in vitro,* which comprises the antibody according to any one of the fifth aspect of the present invention, and the recombinant protein according to the sixth aspect of the present invention, or a combination thereof, used as an active ingredient.

In a thirteenth aspect of the present invention, it provides a method for treating tumors or viral infection related diseases, comprising: using the antibody according to any one of the fifth aspect of the present invention, the recombinant protein according to the sixth aspect of the present invention, or a combination thereof.

In another preferred embodiment, the tumor is cancer.

It should be understood that within the scope of the present invention, the various technical features of the present invention above and the various technical features specifically described hereinafter (as in the embodiments) may be combined with each other to constitute a new or preferred technical solution. Due to space limitations, it is not repeated here.

### Description of the figures

Figure 1 shows the binding activity of CTLA-4-hFc protein and biotin-labeled ligand B7.1-hFc or B7.1-hFc.
Figure 2 shows the FACS detection results of HEK293 cells transfected with CTLA-4 (Y201V) protein.
Figure 3 shows the serum antibody titer of Harbour transgenic mice after HEK293-CTLA-4 cell immunization detected by ELISA.
Figures 4 (A) and (B) show the FACS detection results of the binding reaction of CTLA-4 antibodies and CHOK1-hCTLA-4.
Figures 5 (A) and (B) show the FACS detection results of the binding reaction of CTLA-4 antibodies and 293F-cCTLA-4.
Figures 6 (A), (B) and (C) show the inhibitions of CTLA-4 antibodies on the binding of CTLA-4 protein to its receptor B7.1.
Figures 7 (A), (B) and (C) show the inhibitions of CTLA-4 antibodies on the binding of CTLA-4 protein to its receptor B7.2.
Figure 8 shows the effect of CTLA-4 antibodies on IL-2 secretion in the PBMC lymphocyte stimulation test.
Figures 9 (A) and (B) show the detection results of the binding of antibodies to human CTLA-4 expressing cells by flow cytometry (FACS).
Figures 10 (A) and (B) show the detection results of the binding of antibodies to monkey CTLA-4 expressing cells by flow cytometry (FACS).
Figures 11 (A) and (B) show the inhibition of fully human CTLA-4 antibodies on the binding of CTLA-4 protein to its receptor B7.1.
Figures 12 (A) and (B) show the inhibition of fully human CTLA-4 antibodies on the binding of CTLA-4 protein to its receptor B7.2.
Figure 13 shows the effects of CTLA-4 fully human antibodies on IL-2 secretion in the SEB-dependent PBMC activation test.
Figures 14 (A), (B) (C) and (D) show the effects of CTLA-4 fully human antibodies on IL-2 secretion in the PHA-induced T lymphatic sac cell activation test.
Figure 15 shows the effects of CTLA-4 antibodies on the survival rate of tumor-bearing mice.
Figures 16 (A), (B), (C) and (D) show the determination of the molecular weights of the Fc segments of defucosylated and fucosylated anti-CTLA-4 antibodies by liquid-mass spectrometry.
Figure 17 shows the effects of defucosylated and fucosylated anti-CTLA-4 antibodies on IL-2 secretion in the SEB-dependent PBMC activation test.

### Detailed description

Through extensive and intensive studies, the present inventors used rat-human chimeric antibody-based transgenic mice, which were immunized with 293F-HuCTLA-4 cells (such as recombinantly expressed 293F-HuCTLA4 cells) and obtained highly active fully human antibodies against CTLA-4. Experimental results show that the antibodies have high affinity (K_{D} is 2.15E-09nM) to CTLA-4 proteins of human and monkey, and can inhibit binding of CTLA-4 and its receptors B7.1 and B7.2, and can significantly increase the expression level of IL-2 in human T lymphocytes induced by PHA or human peripheral blood mononuclear cells mediated and activated by SEB, and lack cross-reactivity with human CD28 and other similar protein antigens. The fully human anti-CTLA-4 antibodies obtained have the characteristics of higher affinity and stronger activity *in vitro* and *in vivo.* And the fully human CTLA-4 antibody (such as 97A8D1) has better anti-tumor effects *in vivo* than the antibody Ipilimumab in the prior art. On this basis, the present invention has been completed.

### The terms

### CTLA-4

CTLA-4, the cytotoxic T lymphocyte-associated antigen 4, structurally belongs to the CD28 immunoglobulin superfamily. It is inducibly expressed in activated T cells and constitutively expressed in regulatory T cells. As an immune checkpoint protein, it competes with CD28 for binding the ligands, thereby down-regulating the activity of T cells. Studies on transgenic mice and tumor-bearing mice have shown that CTLA-4 participates in tumor evasion of immune system recognition and killing. Therefore, blocking the activity of CTLA-4 may increase the body's inhibition on tumor growth.

### Antibody

As used herein, the term "antibody" or "immunoglobulin" is a heterotetrameric glycoprotein of about 150,000 daltons with the same structural characteristics, which consists of two identical light chains (L) and two identical heavy chains (H). Each light chain is connected to the heavy chain through a covalent disulfide bond, and the numbers of disulfide bonds between heavy chains of different immunoglobulin isotypes are different. Each heavy and light chain also has regularly spaced intrachain disulfide bonds. Each heavy chain has a variable region (VH) at one end, followed by multiple constant regions. Each light chain has a variable region (VL) at one end and a constant region at the other end; the constant region of the light chain is opposite to the first constant region of the heavy chain, and the variable region of the light chain is opposite to the variable region of the heavy chain. Special amino acid residues form an interface between the variable regions of the light and heavy chains.

As used herein, the term "variable" means that certain parts of the variable region of an antibody differ in sequence, which forms the binding and specificity of various specific antibodies for their specific antigens. However, the variability is not evenly distributed throughout the variable region of the antibody. It is concentrated in three segments called complementary determining regions (CDRs) or hypervariable regions in the light chain and heavy chain variable regions. The more conserved part of the variable region is called the framework region (FR). The variable regions of the natural heavy and light chains each contain four FR regions, which are roughly in the β-folded configuration, connected by the three CDRs that form the connecting loop, and in some cases may form a partly folded structure.β The CDRs in each chain get close through the FR regions and together with the CDRs of the other chain form the antigen-binding site of the antibody (see Kabat et al., NIH Publ. No. 91-3242, Volume I, pages 647-669 (1991)). The constant regions are not directly involved in the binding of antibodies to antigens, but they exhibit different effector functions, such as involved in the antibody-dependent cytotoxicity of antibodies.

The light chains of vertebrate antibodies (immunoglobulins) can be classified into one of two distinct classes (referred to as κ and λ) based on the amino acid sequence of their constant regions. Immunoglobulins can be divided into different types, according to the amino acid sequence of the constant region of the heavy chain. There are five main classes of immunoglobulins: IgA, IgD, IgE, IgG, and IgM, some of which can be further divided into subclasses (isotypes), such as IgG1, IgG2, IgG3, IgG4, IgA, and IgA2. The heavy chain constant regions corresponding to different classes of immunoglobulins are called δ, ε, γ, α, and µ, respectively. The subunit structures and three-dimensional configurations of different classes of immunoglobulins are well known to those skilled in the art.

In general, the antigen-binding properties of an antibody can be described by the three specific regions located in the variable regions of the heavy and light chains, called complementary determining regions (CDR), which divide this segment into 4 framework regions (FR). The amino acid sequences of the four FRs are relatively conservative and do not directly participate in the binding reaction. These CDRs form a circular structure, and get close in space structure through the β sheets formed by the FRs in between. The CDRs on the heavy chain and the CDRs on the corresponding light chain constitute the antigen binding site of the antibody. The amino acid sequences of antibodies of the same type can be compared to determine which amino acids constitute the FR or CDR regions.

The present invention includes not only intact antibodies, but also immunologically active fragments of antibody fragments or fusion proteins formed by antibodies and other sequences. Therefore, the present invention also includes fragments, derivatives and analogs of the antibodies.

In the present invention, antibodies include murine, chimeric, humanized, or fully human antibodies prepared by techniques well known to those skilled in the art. Recombinant antibodies, such as chimeric and humanized monoclonal antibodies, including human and non-human parts, can be obtained by standard DNA recombination techniques, and they are all useful antibodies. A chimeric antibody is a molecule in which different parts come from different animal species, such as a chimeric antibody with a variable region of a monoclonal antibody from a mouse and a constant region from a human immunoglobulin (see, for example, US Patent No. 4,816,567 and US Patent 4,816,397, hereby incorporated by reference in its entirety). Humanized antibodies refer to antibody molecules derived from non-human species, having one or more complementary determining regions (CDRs) derived from non-human species and framework regions derived from human immunoglobulin molecules (see US Patent 5,585,089, hereby incorporated by reference in its entirety). These chimeric and humanized monoclonal antibodies can be prepared using recombinant DNA techniques well known in the art.

In the present invention, the antibody may be monospecific, bispecific, trispecific, or more multispecific.

In the present invention, the antibody of the present invention also includes conservative variants thereof, which means that compared with the amino acid sequence of the antibody of the present invention, there are at most 10, preferably at most 8, more preferably at most 5, most preferably at most 3 amino acids replaced by amino acids with the same or similar properties to form a polypeptide. These conservatively variant polypeptides are preferably produced by amino acid substitution according to Table A.

**Table A**

| Initial residue | Representative substitution | Preferred sub stitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Anti-CTLA-4 antibody

The present invention provides an antibody with high specificity and high affinity against CTLA-4, which comprises a heavy chain and a light chain, wherein the heavy chain contains a heavy chain variable region (VH) amino acid sequence, and the light chain contains a light chain variable region (VL) amino acid sequence.

Preferably, the heavy chain variable region (VH) comprises the following three complementary determining regions or CDRs:
CDR1 shown in SEQ ID NO: 2,
CDR2 shown in SEQ ID NO: 3, and
CDR3 shown in SEQ ID NO: 4;
the light chain variable region (VL) comprises the following three complementary determining regions or CDRs:
CDR1' shown in SEQ ID NO: 6,
CDR2' shown in SEQ ID NO: 7, and
CDR3' shown in SEQ ID NO: 8;
wherein, any one of the above amino acid sequences also includes a derivative sequence that is optionally with at least one amino acid added, deleted, modified, and/or substituted, and is capable of retaining the binding affinity to CTLA-4.

In another preferred embodiment, the sequence with at least one amino acid added, deleted, modified and/or substituted in any of the above amino acid sequences is preferably an amino acid sequence having a homology or sequence identity of at least 80%, preferably at least 85%, more preferably at least 90%, most preferably at least 95% to the above amino acid sequence.

Methods known to those of ordinary skill in the art for determining sequence homology or identity include, but are not limited to: Computational Molecular Biology, Lesk, A.M., Oxford University Press, New York, 1988; Biocomputing: Informatics and Genome Projects, edited by Smith, D.W., Academic Press, New York, 1993; Computer Analysis of Sequence Data, Part I, edited by Griffin, A.M. and Griffin, H.G., Humana Press, New Jersey, 1994; Sequence Analysis in Molecular Biology, von Heinje, G., Academic Press, 1987, and Sequence Analysis Primer, edited by Gribskov, M. and Devereux, J., Stockton Press, New York, 1991, and Carillo, H. and Lipman, D., SIAM J. Applied Math., 48:1073 (1988). The preferred method of determining identity is to obtain the greatest match between the sequences tested. The method of determining identity is compiled in a publicly available computer program. Preferred computer program methods for determining the identity between two sequences include, but are not limited to: GCG package (Devereux, J. et al., 1984), BLASTP, BLASTN, and FASTA (Altschul, S, F. et al., 1990). The BLASTX program is available to the public from NCBI and other sources (BLAST Manual, Altschul, S. et al., NCBI NLM NIH Bethesda, Md. 20894; Altschul, S. et al., 1990). The well-known Smith Waterman algorithm can also be used to determine identity.

The antibody of the present invention may be a double-chain or single-chain antibody, and may be selected from animal-derived antibodies, chimeric antibodies and humanized antibodies, more preferably be selected from humanized antibodies and human-animal chimeric antibodies, more preferably a fully humanized antibody.

The antibody derivatives of the present invention may be single chain antibodies, and/or antibody fragments, such as: Fab, Fab', (Fab')2 or other known antibody derivatives in the art, etc., as well as any one or several of IgA, IgD, IgE, IgG and IgM antibodies or other subtypes.

Wherein, the animal is preferably a mammal, such as a mouse.

The antibody of the present invention may be a chimeric antibody, a humanized antibody, a CDR grafted and/or modified antibody targeting human CTLA-4.

In the above content of the present invention, the number of added, deleted, modified and/or substituted amino acids is preferably not more than 40% of the total number of amino acids in the original amino acid sequence, more preferably not more than 35%, more preferably 1-33%, more preferably 5-30%, more preferably 10-25%, more preferably 15-20%.

In the above content of the present invention, more preferably, the number of added, deleted, modified and/or substituted amino acids may be 1-7, more preferably 1-5, more preferably 1-3, more preferably 1-2.

In another preferred embodiment, the antibody targeting CTLA-4 is 97A8D1, 92C8B6, 31C12F3, 40C4C6 or 97B8E1.

In another preferred embodiment, the amino acid sequence of the heavy chain variable region (VH) of the antibody 97A8D1 is the amino acid sequence shown in SEQ ID NO: 1.

In another preferred example, the amino acid sequence of the light chain variable region of the antibody 97A8D1 is the amino acid sequence shown in SEQ ID NO: 5.

### Preparation of Antibodies

The sequence of the DNA molecule for the antibody or a fragment thereof according to the present invention can be obtained by conventional techniques, for example, methods such as PCR amplification or genomic library screening. In addition, the sequences encoding light chain and heavy chain can be fused together, to form a single-chain antibody.

Once a relevant sequence is obtained, recombination methods can be used to obtain the relevant sequence in large quantities. This is usually carried out by cloning the sequence into a vector, transforming a cell with the vector, and then separating the relevant sequence from the proliferated host cell by conventional methods.

In addition, a relevant sequence can be synthesized artificially, especially when the fragment is short in length. Usually, several small fragments are synthesized first, and then are linked together to obtain a fragment with a long sequence.

It has been possible now to obtain a DNA sequence encoding the antibody (or a fragment thereof, or a derivative thereof) according to the present invention completely by chemical synthesis. Then, the DNA sequence can be introduced into various existing DNA molecules (or, for example, vectors) and cells known in the art. In addition, mutations can also be introduced into the protein sequence according to the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Preferred animal cells include, but are not limited to, CHO-S, HEK-293 cells.

In general, under conditions suitable for expression of the antibody according to the present invention, the host cell obtained is cultured. Then, the antibody according to the present invention is purified by using conventional immunoglobulin purification steps, for example, the conventional separation and purification means well known to those skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained can be identified by conventional means. For example, the binding specificity of a monoclonal antibody can be determined by immunoprecipitation or an *in vitro* binding assay (such as radioimmunoassay (RIA) or enzyme-linked immunosorbent assay (ELISA)). The binding affinity of a monoclonal antibody can be determined by, for example, the Scatchard analysis (Munson et al., Anal. Biochem., 107: 220 (1980)).

The antibody according to the present invention can be expressed in a cell or on the cell membrane, or is secreted extracellularly. If necessary, the recombinant protein can be separated and purified by various separation methods according to its physical, chemical, and other properties. These methods are well known to those skilled in the art. Examples of these methods include, but are not limited to, conventional renaturation treatment, treatment with a protein precipitant (salting out method), centrifugation, osmotic bacteria disruption, ultrasonic treatment, ultracentrifugation, molecular sieve chromatography (gel filtration), adsorption chromatography, ion exchange chromatography, high performance liquid chromatography (HPLC), various other liquid chromatographic techniques, and combinations of these methods.

### Uses

The present invention further provides use of the antibody according to the present invention, for example, for manufacture of a medicament for treating a CTLA-4 related disease. The CTLA-4 related diseases include tumorigenesis, tumor growth and/or metastasis, viral infection and other related diseases.

The uses of the antibody of the present invention include (but not limited to):
(i) Treatment of tumorigenesis, tumor growth and/or metastasis. The tumors include (but are not limited to): breast cancer (such as triple-negative breast cancer), pancreatic cancer, lung cancer (such as non-small cell lung cancer, extensive stage small cell lung cancer, metastatic non-small cell lung cancer), malignant glioma, malignant tumor of digestive organs, stomach cancer, liver cancer, esophageal cancer, kidney cancer, colorectal cancer, metastatic colon cancer, bladder cancer, prostate tumor (such as prostate cancer), endometrial carcinoma, endometrial carcinosarcoma, cervix cancer, ovarian cancer, fallopian tube cancer, leukemia (such as adult acute myeloid leukemia, atypical chronic myelogenous leukemia), bone marrow cancer, sarcoma (such as angiosarcoma, synovial sarcoma), melanoma, recurrent melanoma, mesothelioma, advanced solid cancer, head and neck squamous cell carcinoma, Merkel cell carcinoma, skin T-cell lymphoma, peritoneal tumor, muscle invasiveness bladder cancer, previously treated myelodysplastic syndrome, ovarian epithelial cell carcinoma, urinary system malignancies, adult grade III lymphomatoid granuloma, B-cell chronic lymphocytic leukemia, skin B-cell non-Hodgkin's lymphoma, intraocular lymphoma, choriocarcinoma of testis, neuroblastoma, esophageal cancer. Especially, the tumors include triple-negative breast cancer, pancreatic cancer, malignant glioma and lung cancer, more preferably triple-negative breast cancer and/or pancreatic cancer.
(ii) Treatment of viral infection diseases. The viruses include (but are not limited to): hepatitis B virus, hepatitis C virus (chronic infection), HIV.

### Pharmaceutical Composition

The present invention further provides a composition. In the preferred examples, the composition is a pharmaceutical composition comprising the antibody, or an active fragment, a fusion protein thereof, or a corresponding CAR-T cell, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated. The formulated pharmaceutical composition may be administered by conventional routes, including (but not limited to): intratumoral, intraperitoneal, intravenous, or topical administration.

The pharmaceutical composition according to the present invention can be directly used for binding to a CTLA-4 protein molecule, and thus can be used for preventing and treating diseases such as tumors. In addition, other therapeutic agents can also be used at the same time.

The pharmaceutical composition according to the present invention comprises a safe and effective amount (e.g. 0.001-99 wt %, preferably 0.01-90 wt %, preferably 0.1-80 wt %) of the monoclonal antibody according to the present invention and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The administration amount of an active ingredient is a therapeutically effective amount, for example, about 1 µg per kilogram of body weight to about 5 mg per kilogram of body weight daily. In addition, the polypeptide according to the present invention may also be used in combination with an additional therapeutic agent.

When a pharmaceutical composition is used, a safe and effective amount of antibodies is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 20 mg per kilogram of body weight. Of course, a specific amount should also depend on the factors such as administration route and physical conditions of a patient, which fall into the skills of skilled physicians.

### The main advantages of the invention are:

(1) The transgenic mice used in the present invention can obtain fully human antibodies more easily than wild-type mice, therefore the immunogenicity of antibodies are reduced. And compared with transgenic mice of fully human antibodies, the number of antibodies obtained is larger, and the antibodies have strong affinity, good sequence diversity and high activity.
(2) Compared with antibodies obtained from phage library, the antibody of the present invention obtained by hybridoma technology has high affinity and good sequence expression.
(3) The present invention uses recombinantly expressed 293F-HuCTLA-4 cells. Compared with protein or polypeptide immunogens, the expressed target protein has a more natural conformation; and compared with other recombinantly expressed cells, its expression level is higher.
(4) The present invention obtains antibodies with different sequences, which can specifically bind to CTLA-4 antibodies, and their binding activity level is lower than nanomolar (nM).
(5) The antibodies obtained in the present invention have a good activity in stimulating T cell activation. They can block the binding of CTLA-4 and the two ligands (B7.1 and B7.2) thereof, and can reverse the inhibition of CTLA-4 on T cell activation activity, thereby activating T cells to secrete IL-2.
(6) The antibodies obtained in the present invention exhibit significant activities of inhibiting tumor growth in mice and improving the survival rate of mice (such as human CTLA-4 transgenic mice).
(7) The antibodies obtained in the present invention have a series of excellent characteristics: the variable region sequences have low homologies with the existing antibody (homology <92%), and all activities are better than those from the reference document.

The invention is further illustrated below in conjunction with specific embodiments. It should be understood that the examples are not intended to limit the scope of the invention. The experimental methods without detailed conditions in the following examples are generally in accordance with the conditions described in the conventional conditions such as Sambrook. J et al. "Guide to Molecular Cloning Laboratory" (translated by Huang Peitang et al., Beijing: Science Press, 2002), or in accordance with the conditions recommended by the manufacturer (for example, product manuals). Unless otherwise stated, percentages and parts are calculated by weight. Unless otherwise specified, the experimental materials and reagents used in the following examples are commercially available.

The room temperature described in the examples is a conventional room temperature in the art, and is generally 10-30°C.

Unless otherwise specified, the PBS described in the examples is PBS phosphate buffer, pH 7.2.

### Materials

The H2L2 transgenic mice were obtained (purchased) from Hebo Medicine (Shanghai) Co., Ltd., which could produced a traditional tetrameric antibody consisting of two heavy chains and two light chains (H2L2) with fully human variable regions. The antibodies produced have mature affinity, fully humanized variable regions, and have excellent solubility. Genetically engineered mouse technology is one of the main tools for producing fully human antibodies ^{[4]}.

### General methods

The present invention first prepared human CTLA-4 as an immunogen, and used human antibody transgenic mouse technology to prepare fully human antibodies [Lonberg, et al. 1994, Nature 368(6474): 856-859, Lonberg, N. and Huszar, D. 1995, Intern. Rev. Immunol. 13:65-93, and Harding, F. and Lonberg, N., 1995, Ann. NY Acad. Sci. 764:536-546], and obtained the lead antibody of the CTLA-4 antibody. Then through the preliminary production, purification and verification of the lead antibody, the CTLA-4 antibodies with excellent biological characteristics such as high affinity (affinity KD<1*10⁻⁹M), ability of effectively blocking the binding of CTLA-4 and receptors B7.1 and B7.2, ability of significantly increase the expression levels of IL-2 in human peripheral blood mononuclear cells or T lymphocyte reaction, and lack of cross-reactivity with human CD28 and other similar protein antigens were obtained. Then, the amino acid sequences of the heavy chain variable regions and the light chain variable regions of the CTLA-4 antibodies were obtained using molecular biology sequencing method.

### Example 1 Preparation of CTLA-4 antibody

### (I) Preparation of immunogen A

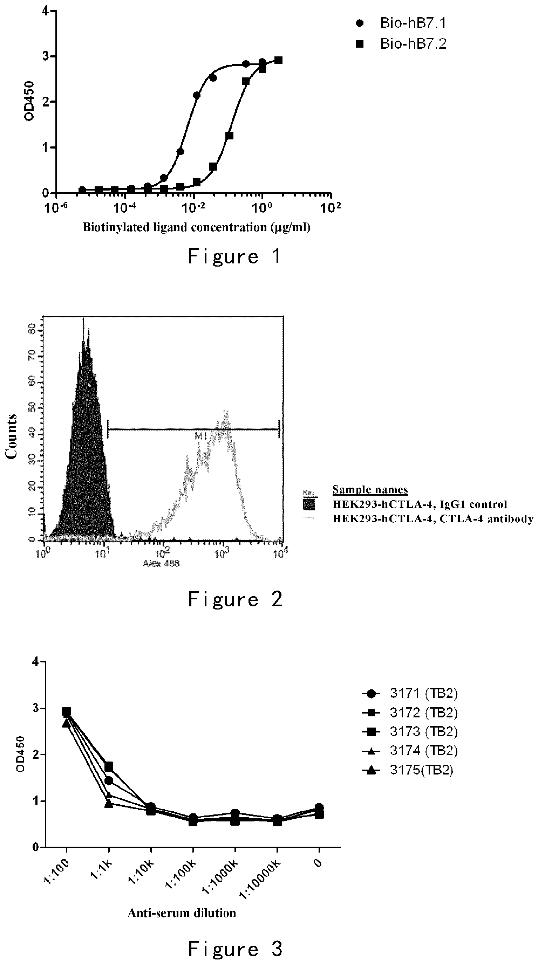

The amino acid sequence Lys36-Asp161 of the extracellular region of human CTLA-4 protein was cloned into the pCpC vector with human IgG Fc fragment (hFc) (purchased from Invitrogen, V044-50) and plasmids were prepared according to the established standard molecular biology methods. For specific methods, see Sambrook, J., Fritsch, E.F., and Maniatis T. (1989). Molecular Cloning: A Laboratory Manual, Second Edition (Plainview, New York: Cold Spring Harbor Laboratory Press). HEK293 cells (purchased from Invitrogen) were transiently transfected (PEI, Polysciences) and expanded using FreeStyle™ 293 (Invitrogen) at 37°C. After 4 days, the cell culture was collected, and the cell components were removed by centrifugation to obtain the culture supernatant containing the extracellular region of CTLA-4 protein. The culture supernatant was loaded onto a protein A affinity chromatography column (Mabselect Sure, purchased from GE Healthcare), and an ultraviolet (UV) detector was used to monitor the change in ultraviolet absorbance (A280nm). After the sample was loaded, the protein affinity chromatography column was washed with PBS phosphate buffer (pH 7.2) until the UV absorption value returned to the baseline, and then eluted with 0.1M glycine hydrochloric acid (pH 2.5). The CTLA-4 protein with hFc tag (CLTA-4-hFc) eluted from the protein A affinity chromatography column was collected and dialyzed overnight with PBS phosphate buffer (pH 7.2) in a refrigerator at 4°C. The dialyzed protein was sterile filtered by a 0.22 micron filter and stored at -80°C after subpackage to obtain purified immunogen A. Immunogen A protein needed a series of quality control tests before use, such as tests of protein concentration, purity, molecular weight and biological activity. The results were shown in Figure 1 and Table 1. Table 1 shows that the binding of CTLA-4 and B7.1 or B7.2 at the protein level varies with the concentration of B7.1 or B7.2. Wherein, the control protein is a non-CTLA-4 fusion protein and the data in the table is OD₄₅₀ₙₘ value.

Wherein, the biological activity of immunogen A was detected by ELISA, specifically:
The hFc-labeled CTLA-4 protein (CTLA-4-hFc, i.e. the immunogen A) was diluted with PBS to 1µg/mL, and added as 100µl/well to the ELISA microplate, and incubated overnight at 4°C. After blocked with ELISA blocking solution (containing 1% BSA, pH 7.4 PBS phosphate buffer solution, wherein the percentage is the mass percentage) at 37°C for two hours, the plated was added with gradient dilution of biotin-labeled B7.1 or B7.2-hFc, and incubated at 37°C for 1 hour. The preparation method of biotin-labeled B7.1-hFc or B7.2-hFc was as follows: reacting B7.1-hFc or B7.2-hFc with a biotinylation reagent. The preparation method of B7.1-hFc or B7.2-hFc was the same as that of immunogen A. Wherein, the amino acid sequence information of B7.1 extracellular domain protein (Val35-Asn242) can be found in number P33681 of Uniprot database; and the amino acid sequence information of B7.2 extracellular domain protein (Ala24-Pro247) can be found in number P42081 of Uniprot database. The biotinylation reagent was purchased from Sigma, and the product number was B3295. For the operating steps of the reaction with the biotinylation reagent, references can be found in the instructions for the biotinylation reagent. The plate was added with streptavidin-labeled horseradish peroxidase (purchased from Sigma, product number S2438) and incubated at room temperature for 30 minutes, and the unbound streptavidin-labeled horseradish peroxidase was washed off with washing solution. And 100µl/well TMB color developing solution was added. After incubated at room temperature for 15 minutes, the plate was added with 50 microliters of IN hydrochloric acid to stop the color reaction, and read with an ELISA plate reader for the OD₄₅₀ₙₘ reading.

**Table 1 Binding activity of CTLA-4-hFc protein and biotin-labeled ligand B7.1-hFc or B7.1-hFc**

| | Coated CTLA-4-hFc, 1ug/ml | | | | | | | | | | |
|---|---|---|---|---|---|---|---|---|---|---|---|
| Biotin labeled ligand, nM | 40 | 13 | 4.4 | 1.5 | 0.49 | 0.16 | 0.06 | 0.02 | 6.1E-03 | 2.0E-03 | 6.8E-04 |
| Biotin labeled B7.1-hFc | 2.65 | 2.69 | 2.72 | 2.65 | 2.74 | 2.7 | 2.36 | 1.15 | 0.44 | 0.23 | 0.18 |
| Biotin labeled B7.2-hFc | 2.72 | 2.81 | 2.84 | 2.58 | 1.31 | 0.45 | 0.19 | 0.19 | 0.16 | 0.14 | 0.15 |

The specific activites are shown in Figure 1 and Table 1, which show that the expressed ligand and receptor proteins had the correct conformations, which were suitable for immunization, establishment of receptor-ligand binding blocking detection methods and identification of antibody activity.

### (II) Preparation of immunogen B

The human CTLA-4 full-length amino acid sequence was mutated to CTLA-4 (Y201V) and cloned into pIRES vector (purchased from Clontech) and the plasmid was prepared. After plasmid transfection on HEK293 cell line and CHOK1 cell line (both purchased from Invitrogen) (transfection was preformed using X-treme GENE HP DNA Transfection Reagent, which was purchased from Roche, Cat #06 366 236 001, and operated according to the instructions), cells were selectively cultured in DMEM medium containing 10% (w/w) FBS and containing 0.5 µg/ml puromycin for 2 weeks. Subcloning was conduted in 96-well culture plate by a limiting dilution method, and the plate was placed at 37° C, 5% (v/v) CO₂. After about 2 weeks, some of the monoclonal wells were selected and amplified into 6-well plates. The amplified clones were stained with known CTLA-4 antibodies and screened by flow cytometry. The monoclonal cell lines with better growth and higher fluorescence intensity were continued to be expanded in culture and cryopreserved in liquid nitrogen, thus obtaing immunogen B. The specific selection results are shown in Table 2 and Figure 2. In Table 2, positive cells (%) refer to the percentage of positive cells number in the total cells number. Figure 2 shows that HEK293 cells have a higher level of CTLA-4 expression, which is suitable for use as an immunogen and for identification of antibody binding activity.

**Table 2 FACS screening and detection results of HEK293 cells transfected with CTLA-4 (Y201V) protein**

| Numbering | Recombinant cell Clone | IgG control | | Anti-CTLA-4 mAb | |
|---|---|---|---|---|---|
| | | Gated (%) | MFI | Gated (%) | MFI |
| 1 | 4-2C3 | 3.5 | 5.2 | 99.1 | 545.6 |
| 2 | 4-1E10 | 4.0 | 5.2 | 99.2 | 545.5 |
| 3 | 4-1C5 | 2.9 | 5.1 | 99.7 | 504.5 |
| 4 | 4-2D2 | 3.8 | 5.1 | 99.1 | 424.9 |

### (III) Preparation of hybridoma cells and screening of antibody

Harbour transgenic mice are introduced with human immunoglobulin variable region genes and rat immunoglobulin constant region genes, while the Ig expression of the mice own is silenced (F.G. Franklin, et al, patent #WO 2010/070263 A1). The transgenic mice can produce immune response and antibody titer equivalent to that produced by normal mice (such as Balb/c) after being immunized with antigen.
A. 6-8 weeks old Harbour human antibody transgenic mice (purchased from Beijing Weitong Lihua Company) were used for immunization by immunogen A, and the mice were raised under SPF conditions. During the initial immunization, immunogen A protein was emulsified with Freund's complete adjuvant and injected intraperitoneally with 0.25 ml, that is, 100 micrograms of immunogen A protein was injected per mouse. During the boosting immunization, immunogen A protein was emulsified with Freund's incomplete adjuvant and injected intraperitoneally with 0.25 ml, that is, 50 micrograms of immunogen A protein was injected per mouse. The interval between the initial immunization and the first boosting immunization was 2 weeks. After that, the intervals between each boosting immunization were 3 weeks. Blood was collected 1 week after each boosting immunization, and the antibody titer and specificity of immunogen A in the serum were detected by ELISA and FACS. The results are shown in Table 3 and Figure 3. Table 3 shows that the serum of mice immunized with CTLA-4-hFc had different degrees of binding to the immunogen, showing antigen-antibody response, and the highest dilution was about one thousand (1000). Wherein, the blank control was 1% (w/w) BSA, and the batch referred to the mice serum on the seventh day after the third boosting immunization. The data in the table is the value of OD₄₅₀ₙₘ.
B. 6-8 weeks old Harbour human antibody transgenic mice (purchased from Beijing Weitong Lihua Company) were used for immunization by immunogen B, and the mice were raised under SPF conditions. The HEK293-hCTLA-4 (Y201V) stable cell line containing human CTLA-4 obtained in step (II) was expanded to a 90% confluence in a T-75 cell culture flask, and the medium was exhausted. Cells were washed with DMEM basal medium (purchased from Invitrogen) twice, and then treated with enzyme-free cell dissociation solution (purchased from Invitrogen) at 37°C until the cells were detached from the wall of the culture dish, and then the cells were collected. Cells were washed twice with DMEM basal medium and counted, and then diluted with phosphate buffer (pH 7.2) to 2×10⁷ cells per ml. Each mouse was intraperitoneally injected with 0.5 ml of cell suspension during each immunization. The interval between the first and the second immunization was 2 weeks. After that, the intervals between each subsequent immunization were 3 weeks. Except for the initial immunization, blood was collected one week after each immunization, and the antibody titer and specificity in the serum were detected by ELISA. Table 3 and Figure 3 show the results of the antibody titer in serum detected by ELISA after HEK-CTLA-4 cell immunization.

**Table 3 Serum antibody titer of Harbour transgenic mice after HEK293-CTLA-4 cell immunization detected by ELISA**

| **OD₄₅₀ₙₘ** | **Serum dilution** | | | | | | |
|---|---|---|---|---|---|---|---|
| **batch** | 1:100 | 1:10³ | 1:10⁴ | 1:10⁵ | 1:10⁶ | 1:10⁷ | Blank control |
| 3171(TB3) | 2.91 | 1.44 | 0.88 | 0.64 | 0.74 | 0.62 | 0.86 |
| 3172(TB3) | 2.91 | 1.71 | 0.83 | 0.59 | 0.65 | 0.58 | 0.81 |
| 3173(TB3) | 2.93 | 1.75 | 0.79 | 0.6 | 0.57 | 0.6 | 0.71 |
| 3174(TB3) | 2.87 | 1.13 | 0.83 | 0.59 | 0.63 | 0.56 | 0.84 |
| 3175(TB3) | 2.68 | 0.95 | 0.79 | 0.56 | 0.62 | 0.56 | 0.73 |

Usually, after 3 times of immunization with the immunogen A or immunogen B, most mice had an ELISA titer of more than 1:1000, indicating that H2L2 mice can have a better humoral immune response to the immunogen, and their spleen cells can be used for Hybridoma cell preparation.

Before the completion of step A or B, each selected mouse was intraperitoneally injected with 100 micrograms of purified CTLA-4-hFc (for mice that had been immunized with immunogen A and immunogen B) or HEK293-hCTLA-4 stable cells containing human CTLA-4 (for mice that had been immunized with immunogen B). Mice were sacrificed 5 days later, and spleen cells were collected. NH₄OH was added to a final concentration of 1% (w/w) to lyse the mixed red blood cells in the spleen cells to obtain a spleen cell suspension. Cells were washed 3 times by centrifugation at 1000 revolutions per minute in DMEM basal medium, and then mixed with mouse myeloma cells SP2/0 (purchased from ATCC) at a ratio of 5:1 according to the number of viable cells. High-efficiency electrofusion or PEG method (see METHODS IN ENZYMOLOGY, VOL. 220) was used for cell fusion. The fused cells were diluted into DMEM medium containing 20% fetal bovine serum and 1×HAT, wherein the percentage was the mass percentage. Then the cell solution was added as 1×10⁵/20 microliters per well to a 96-well cell culture plate, and put in a 5% CO₂, 37°C incubator, wherein the percentage was the volume percentage. After 14 days, ELISA and Acumen (microwell plate cell detection method) were used to screen the supernatants in cell fusion plate. The positive clones with OD₄₅₀ₙₘ>1.0 in ELISA and MFI value>100 in Acumen were expanded to a 24-well plate, in the medium of DMEM (Invitrogen) containing 10% (w/w) of fetal bovine serum, and cultured at 37°C and 5% (v/v) CO₂. After 3 days of culture, the culture solution expanded in the 24-well plate was centrifuged. The supernatant was collected, and the supernatant was analyzed for antibody subtypes. ELISA and FACS were used to determine the binding activity to CTLA-4 protein and CTLA-4 positive cells, and the ligand receptor binding experiment was used to determine the blocking activity of the antibody sample to the CTLA-4 receptor.

According to the results of the 24-well plate screening, hybridoma cells with OD₄₅₀ₙₘ>1.0 in the ELISA experiment, MFI value>50 in the FACS experiment, and the blocking inhibition rate of the hybridoma cell culture supernatant on CTLA-4 receptor reaching 60% in the ligand receptor binding experiment were selected as qualified positive clones. The qualified hybridoma cells were selected to subclone in a 96-well plate by limiting dilution method, and cultured in a DMEM medium (purchased from Invitrogen) containing 10% (w/w) FBS, at 37°C, 5% (v/v) CO₂. 10 days after subcloning, ELISA and Acumen were used for preliminary screening, and positive monoclones were selected and amplified to a 24-well plate to continue culture. After 3 days, FACS was used to determine the positive antigen binding and the CTLA-4 receptor ligand binding experiment was used to evaluate the biological activity (the evaluation criteria were OD₄₅₀ₙₘ>1.0 in the ELISA experiment, MFI value>50 in the FACS experiment, and the blocking inhibition rate of the hybridoma cell culture supernatant on B7.1 ligand reached 60% in the ligand receptor binding experiment).

According to the test results of the 24-well plate samples, the positive clones were expanded in DMEM (purchased from Invitrogen) medium containing 10% (w/w) FBS at 37°C and 5% (v/v) CO₂. The cells were suspended in freezing solution [DMEM containing 20% (w/w) FBS and 10% (w/w) DMSO], and cryopreserved in liquid nitrogen according to conventional methods, to obtain hybridoma cells of the present invention, which can be used for subsequent antibody production, purification and amino acid sequence determination.

### Example 2 Identification of chimeric antibodies

### (I) Detection of the binding of antibodies to CTLA-4 expressing cells by Flow cytometry (FACS)

The pIRES plasmid containing the full-length nucleotide sequence encoding human CTLA-4 described in the step (II) in Example 1 was transfected into a 293F cell line to obtain a stable 293F cell line containing human CTLA-4 (herein referred to as HEK293-hCTLA-4 stable cell line). The pIRES plasmid with the monkey-derived CTLA-4 full-length gene was transfected into the HEK293 cell line to construct a HEK293 stable cell line containing monkey CTLA-4 (herein referred to as HEK293-cCTLA-4 stable cell line), wherein the database accession number of the monkey CTLA-4 nucleotide sequence is XM_005574014.1. The HEK293-hCTLA-4 stable cell line and HEK293-cCTLA-4 stable cell line were expanded in a T-75 cell culture flask to a 90% confluence. The medium was aspirated, and the cells were washed with HBSS buffer (Hanks Balanced Salt Solution, purchased from Invitrogen) twice, then treated with an enzyme-free cell dissociation fluid (Versene solution, purchased from Life technology) and the cells were collected. The cells were washed with HBSS buffer twice. After counted, the cells were diluted with HBSS buffer to 2×10⁶ cells per ml, added with 1% goat serum blocking solution, wherein the percentage was the mass percantage. Cells were incubated on ice for 30 minutes, then washed twice with HBSS buffer by centrifugation. The collected cells were suspended in the FACS buffer (HBSS containing 1% BSA, wherein the percentage was the mass percantage) to 2×10⁶ cells/ml, added as 100 microliters per well to a 96-well FACS reaction plate, added with 100 microliters per well of the purified CTLA-4 antibody sample to be tested, incubated on ice for 2 hours. The plate was washed twice with the FACS buffer by centrifugation, added with 100 microliters of fluorescent (Alexa 488)-labeled secondary antibodies (purchased from Invitrogen) per well, and incubated on ice for 1 hour. The plate was washed 3 times with FACS buffer by centrifugation, added with 100µl fixative solution [4% (v/v) Paraformaldehyde] per well to suspend the cells. 10 minutes later, it was washed 2 times with FACS buffer by centrifugation. The cells were suspended with 100 microliters of FACS buffer, FACS (FACS Calibur, purchased from BD) was used for detection and the results were analyzed.

**Table 4 FACS detection results of the binding reaction of CTLA-4 antibodies and CHOK1-hCTLA-4**

| Clone ID | Mean fluorescence intensity | | | |
|---|---|---|---|---|
| | Antibody concentration (nM) | | | |
| | 66.67 | 6.67 | 0.67 | 0.07 |
| 97A8D1 | 617 | 395 | 116 | 20 |
| 92C8B6 | 603 | 420 | 154 | 29 |
| 31C12F3 | 469 | 386 | 95 | 27.6 |
| 40C4C6 | 449 | 403 | 146 | 43.3 |
| 97B8E1 | 606 | 347 | 125 | 22 |
| IgG control | 8 | 3 | 2 | 2 |

**Table 5 FACS detection results of the binding reaction of CTLA-4 antibodies and 293F-cCTLA-4**

| Clone ID | Mean fluorescence intensity | | | | | | | |
|---|---|---|---|---|---|---|---|---|
| | Antibody concentration (nM) | | | | | | | |
| | 200 | 40 | 8 | 1.6 | 0.32 | 0.064 | 0.0128 | 0.000128 |
| hIgG1 | 4.9 | 3.3 | 2.9 | 2.6 | 2.4 | 2.6 | 2.6 | 2.7 |
| 40C4C6 | 643.1 | 625.6 | 618.8 | 421.2 | 218.6 | 68.6 | 23.0 | 2.4 |
| 31C12F3 | 667.9 | 711.7 | 653.8 | 454.9 | 260.8 | 97.7 | 34.3 | 2.4 |
| 92C8B6 | 711.1 | 800.1 | 721.9 | 465.1 | 208.1 | 67.7 | 22.2 | 2.3 |
| 97A8D1 | 830.7 | 910.0 | 753.8 | 513.1 | 225.4 | 67.2 | 23.1 | 2.4 |
| 97B8E1 | 783.7 | 810.0 | 756.7 | 504.1 | 269.9 | 96.8 | 35.7 | 2.3 |

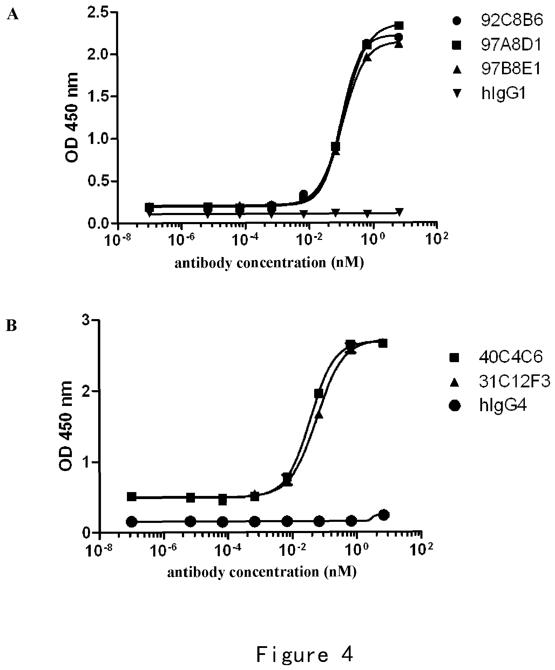

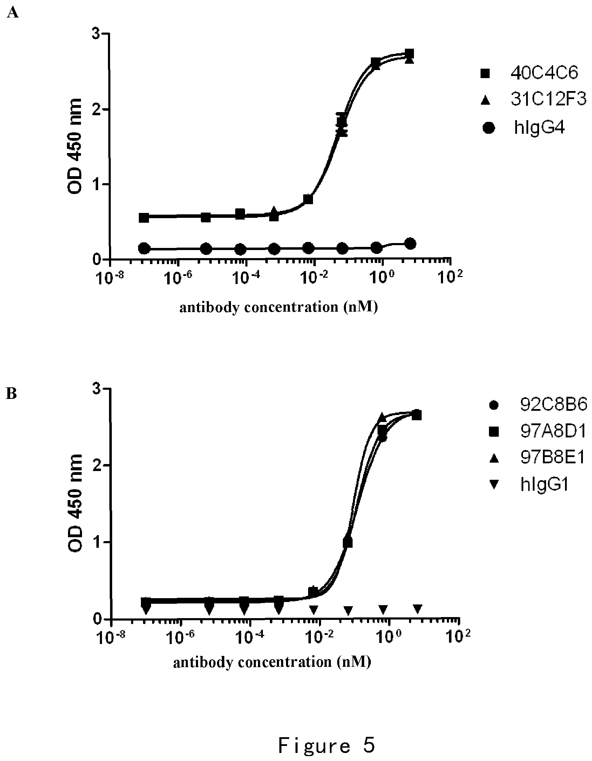

The results are shown in Figure 4 and Figure 5, Table 4 and Table 5. The antibody to be tested can bind to human or monkey CTLA-4 proteins on the cell surface. The activity of each antibody was equivalent, indicating that binding abilities of the antibodies to CTLA-4 were strong. Wherein, the IgG control was human IgG, and the data in the table is the average fluorescence intensity values of the cell populations measured by MFI.

### (II) Detection of the blocking effect of CTLA-4 antibodies on the binding of CTLA-4 protein to its receptor B7.1 or B7.2

CTLA-4 receptor ligand binding assay was used for detection of the blocking effect of CTLA-4 antibodies on the binding of CTLA-4 protein to its receptor B7.1 or B7.2.

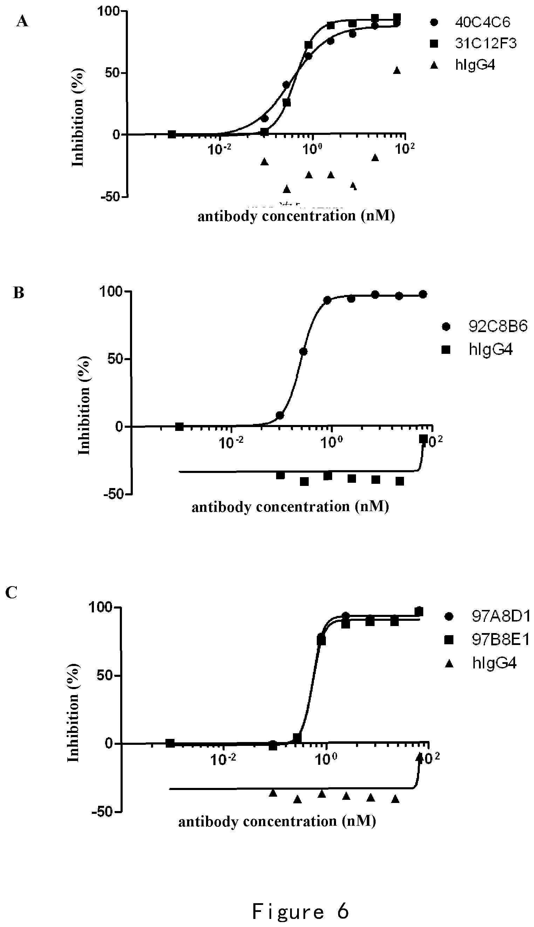

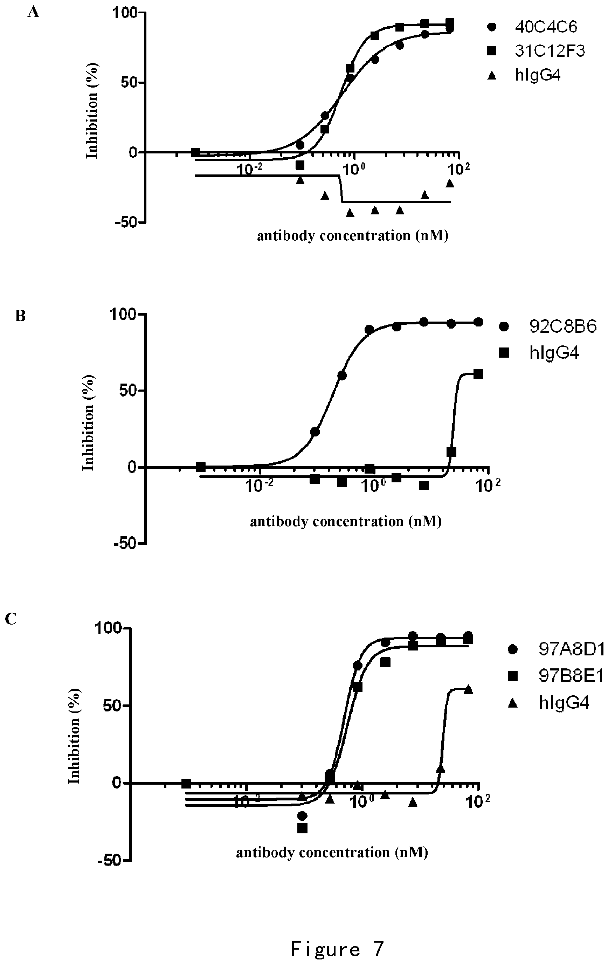

CTLA-4 extracellular domain protein (CTLA-4-hFc) was diluted with PBS to a final concentration of 1.0 µg/mL, and then added to a 96-well ELISA plate as 100 µl per well. The plate was covered with a plastic film and incubated overnight at 4 °C. The plate was washed twice with plate washing solution [PBS containing 0.01% (v/v) Tween 20] on the next day, and added with blocking solution [PBS containing 0.01% (v/v) Tween 20 and 1% (w/w) BSA] and blocked at room temperature for 2 hours. The blocking solution was discarded, and the plate was added with 50µl of the purified CTLA-4 antibody sample to be tested obtained in Example 2 to each well, and then added with biotin-labeled B7.1 extracellular domain protein (B7.1-hFc) or B7.2-hFc, 100 microliters per well, mixed well and incubated at 37°C. After 2 hours, the plate was washed 3 times with a plate washing solution [PBS containing 0.01% (v/v) Tween 20]. HRP (horseradish peroxidase) labeled avidin diluent (purchased from Sigma) was added 100 microliters per well, and after the plate was incubated for 2 hours at 37°C, it was washed with plate washing solution [PBS containing 0.01% (v/v) Tween 20] for 3 times. 100µl of TMB substrate was added to each well. After incubated at room temperature for 30 minutes, the plate was added with 100µl of stop solution (1.0N HCl) to each well. An ELISA plate reader (SpectraMax 384plus, Molecular Device) was used to read the A450nm value. The results were shown in Figure 6, Figure 7 and Table 6, Table 7.

**Table 6 The inhibition of the CTLA-4 antibodies on the binding of CTLA-4 protein to its receptor B7.1**

| Inhibition rate (%) | Antibody concentration (nM) | | | | | | | |
|---|---|---|---|---|---|---|---|---|
| Clone number | 66.7 | 22.2 | 7.4 | 2.5 | 0.823 | 0.274 | 0.091 | 0.00091 |
| 40C4C6 | 89.7 | 87.5 | 80.7 | 75.2 | 63.1 | 40.0 | 12.7 | 0 |
| 31C12F3 | 94.4 | 93.6 | 89.6 | 87.7 | 72.2 | 25.7 | 1.7 | 0 |
| 92C8B6 | 97.0 | 96.0 | 97.0 | 94.0 | 93.0 | 55.0 | 8.0 | 0.0 |
| 97A8D1 | 97.0 | 91.0 | 91.0 | 93.0 | 78.0 | 4.0 | -1.0 | 0.0 |
| 97B8E1 | 96.0 | 89.0 | 89.0 | 87.0 | 75.0 | 4.0 | -2.0 | 0.0 |
| hIgG4 | -10.0 | -41.0 | -40.0 | -39.0 | -37.0 | -41.0 | -36.0 | 0.0 |

**Table 7 The inhibition of the CTLA-4 antibodies on the binding of CTLA-4 protein to its receptor B7.2**

| Inhibition rate (%)) | Antibody concentration (nM) | | | | | | | |
|---|---|---|---|---|---|---|---|---|
| Clone number | 66.7 | 22.2 | 7.4 | 2.5 | 0.823 | 0.274 | 0.091 | 0.00091 |
| 40C4C6 | 88.7 | 84.6 | 76.6 | 66.6 | 53.3 | 26.4 | 5.3 | 0 |
| 31C12F3 | 92.8 | 92.1 | 89.9 | 83.6 | 60.2 | 17.1 | -8.8 | 0 |
| 92C8B6 | 95 | 94 | 95 | 92 | 90 | 60 | 23 | 0 |
| 97A8D1 | 95 | 94 | 95 | 91 | 76 | 6 | -21 | 0 |
| 97B8E1 | 93 | 91 | 89 | 78 | 62 | 2 | -29 | 0 |
| hIgG4 | 61 | 10 | -12 | -7 | -1 | -10 | -8 | 0 |

The results show that the obtained antibodies can inhibit the binding of CTLA-4 protein and its ligand B7.1 or B7.2 to varying degrees, and the activities of the tested antibodies were comparable. Wherein, the IgG control is human IgG, and the data in the table is the inhibition rate (%).

### (III) Detection of the effect of CTLA-4 antibody on lymphocyte activity by lymphocyte stimulation test

In the lymphocyte stimulation test, CTLA-4 antibodies block the binding of CTLA-4 protein and its receptor B7.1 or B7.2 to relieve the inhibition of T lymphocyte activity, thereby stimulating the proliferation of T cells.

### 1. Isolation of Peripheral blood mononuclear lymphocyte PBMCs from the whole blood using Ficoll

The freshly obtained whole blood was diluted with phosphate bufferPBS at a volume ratio of 1:1, to obtain diluted whole blood. A sterile pipette was used to gently spread the diluted whole blood on the surface of Ficoll (purchased from GE Healthcare). The volume ratio of Ficoll to diluted whole blood was 3:4. The solution was mixed without shaking, gradiently centrifuged at 400g at room temperature 20° C for 30 minutes. The solution in the centrifuge tube after centrifugation was divided into three layers, wherein the upper layer was plasma and the middle layer was milky white, which was mononuclear lymphocytes. A sterile pipette was used to gently aspirate the middle layer cells, collected into a new centrifuge tube, diluted to three times of volume with PBS phosphate buffer, and centrifuged at 100g at room temperature for 10 minutes, then the suprenatant was discarded. The lymphocytes were resuspended to 10mL in PBS phosphate buffer, and the previous steps were repeated to remove the platelets. Finally, the lymphocytes were resuspended in 10 mL of multi-component RPMI1640 medium (purchased from Invitrogen) containing 10% fetal bovine serum for use, namely peripheral blood mononuclear lymphocytes PBMCs, and the percentages were mass percentages.

### 2. SEB-dependent PBMC stimulation experiment

Before the test, the purified CTLA-4 antibody obtained in Example 2 diluted in equal volume ratio was prepared to obtain the sample solution to be tested.

The obtained peripheral blood mononuclear lymphocytes PBMCs were plated with 1×10⁵ cells, 100 microliters per well, on a 96-well cell culture plate, and then the test sample solution was added to the culture plate and incubated at room temperature for 30 minutes. Finally, the superantigen SEB was added. Each reaction well contained 50 microliters of 100ng/ml SEB. The volume of each reaction well was 250µL. The reaction plate was incubated in a 37°C, 5% CO₂ incubator for 72 hours, and then the supernatant was collected. The supernatant was frozen at -20°C, and the percentage was the volume percentage.

### 3. Detection of cytokine interleukin IL-2 enzyme-linked immunosorbent assay in cell supernatant

In the detection of cytokine interleukin IL-2 enzyme-linked immunosorbent assay in the cell supernatant, the R&D system related detection kit Quantikine ELISA human IL-2 (S2050) was used, and operated in accordance with the instructions. All test reagents except the tested antibodies were provided by the test kit.

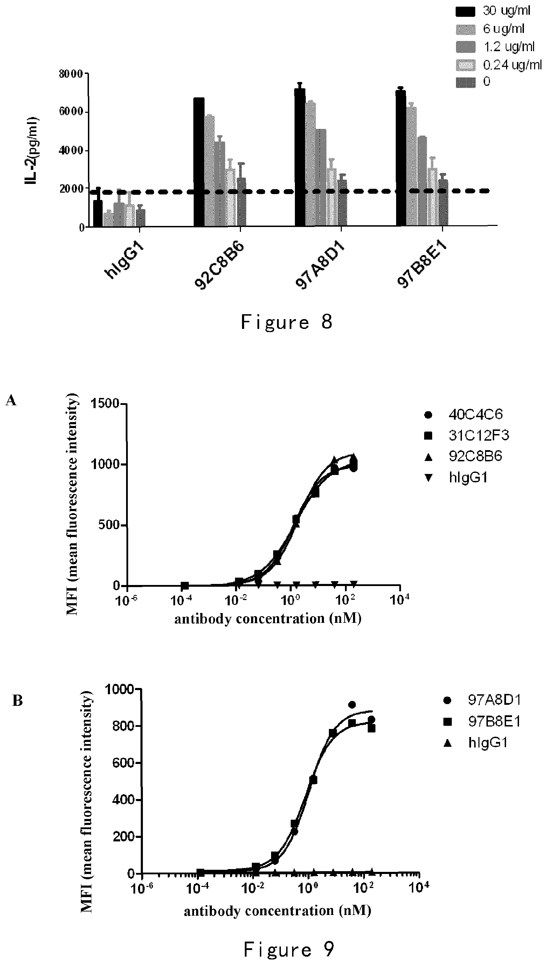

The enzyme-linked immunosorbent assay to determine the cytokine interleukin IL-2 content in the cell supernatant used a double antibody sandwich ELISA kit (purchased from R&D Systems, IL-2 Cat # S2050). The experimental operation was strictly in accordance with the requirements of the kit instructions, and all test reagents were provided by the kit. The specific experiment is briefly described as follows. The IL-2 polyclonal antibody was coated on an ELISA microtiter plate, and the cell supernatant obtained in step II was used as the test sample. The standard and the test sample were incubated at room temperature for 2 hours. 400 microliters of washing solution was added to each well, the plate washing was repeated 4 times. Then horseradish peroxidase-labeled antibody against human IL-2 was added, and incubated for 2 hours at room temperature to form an immune complex with IL-2 on the microplate and the microwells were washed. The substrate was added for color development, protected from light at room temperature for 30 minutes. Finally the stop solution was added, and the absorbance at A450nm was measured with a microplate reader. The effects of CTLA-4 antibodies on IL-2 secretion in the PBMC stimulation experiment described in step 2 were detected. The results are shown in Figure 8 and Table 8.

**Table 8 The effect of CTLA-4 antibodies on IL-2 secretion in the PBMC lymphocyte stimulation test**

| Clone ID | IL-2 (pg/mL) | | | | |
|---|---|---|---|---|---|
| | Antibody concentration (ug/mL) | | | | |
| | 10 | 2 | 4 | 0.08 | 0.016 |
| 97A8D1 | 7979.7 | 4947.6 | 1523.1 | 742.1 | 336.3 |
| 92C8B6 | 6119.7 | 3737.2 | 1418.8 | 830.5 | 355.1 |
| 97B8E1 | 7911.4 | 4449.5 | 1947.4 | 823.6 | 446.1 |
| IgG control | Beyond the range | 102.1 | 343.0 | 222.9 | 178.0 |

The results show that the antibodies to be tested can enhance the IL-2 secretion of PBMC in the PBMC lymphocyte stimulation test, and the activation effects were concentration gradient dependent, and the activity rate of 97A8D1 was better than those of other antibodies. Wherein, the hIgG control was human IgG, and the data in the table is the IL-2 value (pg/mL).

### Example 3 Determination of amino acid sequences of light and heavy chain variable regions

Isolation of total RNA: After the supernatant obtained from the subclonal culture of Example 1 was tested for antigen binding (that is, after the verification and activity determination in Example 3-6), 5×10⁷ hybridoma cells were collected by centrifugation, added with 1mL Trizol and mixed well and transferred to a 1.5mL centrifuge tube, and allowed to stand at room temperature for 5 minutes. The tube was added with 0.2mL chloroform, shaked for 15 seconds, let stand for 2 minutes, and centrifuged at 12000g at 4°C for 5 minutes. The supernatant was taken and transferred to a new 1.5mL centrifuge tube. 0.5 mL of isopropanol was added, and the liquid in the tube was gently mixed, and let stand at room temperature for 10 minutes. After centrifuged at 12000g for 15 minutes at 4°C, the supernatant was discarded. 1 mL of 75% ethanol (the percentage was volume percentage) was added, and the precipitate was gently washed, centrifuged at 12000g at 4°C for 5 minutes. The supernatant was discarded, and the precipitate was dried, and added with DEPC-treated H₂O for dissolution (55°C water bath to promote dissolution for 10 minutes). The total RNA was obtained.

Reverse transcription and PCR: 1µg of total RNA was taken, and a 20ul system was configured, added with reverse transcriptase and reacted at 42°C for 60 minutes, and the reaction was terminated at 7° C for 10 minutes. 50µl PCR system was configured, comprising 1µl cDNA, 25pmol of each primer, 1µl DNA polymerase and a matching buffer system, 250µmol dNTPs. PCR program was set, comprising pre-denaturation 95° C 3min, denaturation 95° C 30s, annealing 55° C 30s, extension 72° C 35s, and further extension at 72° C for 5 min after 35 cycles. And the PCR product was obtained. The kit used for reverse transcription was PrimeScript RT Master Mix, purchased from Takara, and the catalog number was RR036; the kit used for PCR was Q5 ultra-fidelity enzyme, purchased from NEB, and the catalog number was M0492.

Cloning and sequencing: 5µl of PCR product was taken for agarose gel electrophoresis detection, and the column recovery kit was used to purify the positive samples. Wherein, the recovery kit was NucleoSpin® Gel & PCR Clean-up, purchased from MACHEREY-NAGEL, and the catalog number was 740609. Ligation reaction was performed in a 10µl of reaction system containing sample 50ng, T vector 50ng, ligase 0.5µl, buffer 1µl, and reacted for half an hour at 16°C to obtain the ligation product. Wherein, the ligation kit was T4 DNA ligase, purchased from NEB, and the catalog number was M0402. 5µl of ligation product was taken and added into 100µl of competent cells (Ecos 101competent cells, purchased from Yeastern, catalog number FYE607) in ice bath for 5 minutes. Then heat shock was carrited out in a 42°C water bath for 1 minute, and put back on ice for 1 minute, added with 650µl of antibiotic-free SOC medium, resuscitated on a 37°C shaker at 200RPM for 30 minutes. 200µl of the culture was taken and spreaded on LB solid medium containing antibiotics and incubated overnight at 37°C in an incubator. The next day, the primers M13F and M13R on the T vector were used to configure a 30µl PCR system to perform colony PCR. A pipette tip was used to dip the colony into the PCR reaction system and pipette, and 0.5µl was aspirated onto another piece of 100nM ampicillin LB solid petri dish to save the strain. After the PCR reaction, 5µl was taken out for agarose gel electrophoresis detection, and the positive samples were sequenced. Wherein, the steps of sequencing can be found in Kabat, Sequences of Proteins of Immunological Interest, National Institutes of Health, Bethesda, Md. (1991).

The sequencing results are shown in Table 9.

**Table 9 CTLA-4 antibody gene sequence numbers**

| Clone number | Heavy chain protein variable region | Light chain protein variable region |
|---|---|---|
| 97A8D1 | **41** | **42** |
| | (CDR1: nt 88-105) | (CDR1: nt 73-108) |
| | (CDR2: nt 148-198) | (CDR2: nt 151-177) |
| | (CDR3: nt 289-321) | (CDR3: nt 268-294) |
| 92C8B6 | **43** | **44** |
| | (CDR1: nt 88-105) | (CDR1: nt 73-105) |
| | (CDR2: nt 148-198) | (CDR2: nt 148-174) |
| | (CDR3: nt 289-327) | (CDR3: nt 265-294) |
| 31C12F3 | **45** | **46** |
| | (CDR1: nt 88-105) | (CDR1: nt 73-105) |
| | (CDR2: nt 148-198) | (CDR2: nt 148-174) |
| | (CDR3: nt 286-327) | (CDR3: nt 265-291) |
| 40C4C6 | **47** | **48** |
| | (CDR1: nt 88-105) | (CDR1: nt 73-105) |
| | (CDR2: nt 148-198) | (CDR2: nt 148-174) |
| | (CDR3: nt 289-327) | (CDR3: nt 265-291) |
| 97B8E1 | **49** | **50** |
| | (CDR1: nt 88-105) | (CDR1: nt 73-105) |
| | (CDR2: nt 148-198) | (CDR2: nt 148-174) |
| | (CDR3: nt 289-330) | (CDR3: nt 265-291) |

Wherein, the numbers in Table 9 are the "SEQ ID NO:" in the sequence listing. For example, the nucleotide sequence encoding the variable region of the heavy chain protein of 97A8D1 is SEQ ID NO: 41 in the sequence listing.

Wherein, the nucleotide sequence encoding the CDR1 region in the variable region of the heavy chain protein of 97A8D1 is from position 88 to position 105 in SEQ ID NO: 41 of the sequence listing;
the nucleotide sequence encoding the CDR2 region in the variable region of the heavy chain protein of 97A8D1 is from position 148 to position 198 in SEQ ID NO: 41 of the sequence listing;
the nucleotide sequence encoding the CDR3 region in the variable region of the heavy chain protein of 97A8D1 is from position 289 to position 321 in SEQ ID NO: 41 of the sequence listing;
the nucleotide sequence encoding the CDR1 region in the variable region of the light chain protein of 97A8D1 is from position 73 to position 108 in SEQ ID NO: 42 of the sequence listing;
the nucleotide sequence encoding the CDR2 region in the variable region of the light chain protein of 97A8D1 is from position 151 to position 177 in SEQ ID NO: 42 of the sequence listing;
the nucleotide sequence encoding the CDR3 region in the variable region of the light chain protein of 97A8D1 is from position 268 to position 294 in SEQ ID NO: 42 of the sequence listing;
the nucleotide sequence encoding the CDR1 region in the variable region of the heavy chain protein of 92C8B6 is from position 88 to position 105 in SEQ ID NO: 43 of the sequence listing;
the nucleotide sequence encoding the CDR2 region in the variable region of the heavy chain protein of 92C8B6 is from position 148 to position 198 in SEQ ID NO: 43 of the sequence listing;
the nucleotide sequence encoding the CDR3 region in the variable region of the heavy chain protein of 92C8B6 is from position 289 to position 327 in SEQ ID NO: 43 of the sequence listing;
the nucleotide sequence encoding the CDR1 region in the variable region of the light chain protein of 92C8B6 is from position 73 to position 105 in SEQ ID NO: 44 of the sequence listing;
the nucleotide sequence encoding the CDR2 region in the variable region of the light chain protein of 92C8B6 is from position 148 to position 174 in SEQ ID NO: 44 of the sequence listing;
the nucleotide sequence encoding the CDR3 region in the variable region of the light chain protein of 92C8B6 is from position 265 to position 294 in SEQ ID NO: 44 of the sequence listing;
the nucleotide sequence encoding the CDR1 region in the variable region of the heavy chain protein of 31C12F3 is from position 88 to position 105 in SEQ ID NO: 45 of the sequence listing;
the nucleotide sequence encoding the CDR2 region in the variable region of the heavy chain protein of 31C12F3 is from position 148 to position 198 in SEQ ID NO: 45 of the sequence listing;
the nucleotide sequence encoding the CDR3 region in the variable region of the heavy chain protein of 31C12F3 is from position 286 to position 327 in SEQ ID NO: 45 of the sequence listing;
the nucleotide sequence encoding the CDR1 region in the variable region of the light chain protein of 31C12F3 is from position 73 to position 105 in SEQ ID NO: 46 of the sequence listing;
the nucleotide sequence encoding the CDR2 region in the variable region of the light chain protein of 31C12F3 is from position 148 to position 174 in SEQ ID NO: 46 of the sequence listing;
the nucleotide sequence encoding the CDR3 region in the variable region of the light chain protein of 31C12F3 is from position 265 to position 291 in SEQ ID NO: 46 of the sequence listing;
the nucleotide sequence encoding the CDR1 region in the variable region of the heavy chain protein of 40C4C6 is from position 88 to position 105 in SEQ ID NO: 47 of the sequence listing;
the nucleotide sequence encoding the CDR2 region in the variable region of the heavy chain protein of 40C4C6 is from position 148 to position 198 in SEQ ID NO: 47 of the sequence listing;
the nucleotide sequence encoding the CDR3 region in the variable region of the heavy chain protein of 40C4C6 is from position 289 to position 327 in SEQ ID NO: 47 of the sequence listing;
the nucleotide sequence encoding the CDR1 region in the variable region of the light chain protein of 40C4C6 is from position 73 to position 105 in SEQ ID NO: 48 of the sequence listing;
the nucleotide sequence encoding the CDR2 region in the variable region of the light chain protein of 40C4C6 is from position 148 to position 174 in SEQ ID NO: 48 of the sequence listing;
the nucleotide sequence encoding the CDR3 region in the variable region of the light chain protein of 40C4C6 is from position 265 to position 291 in SEQ ID NO: 48 of the sequence listing;
the nucleotide sequence encoding the CDR1 region in the variable region of the heavy chain protein of 97B8E1 is from position 88 to position 105 in SEQ ID NO: 49 of the sequence listing;
the nucleotide sequence encoding the CDR2 region in the variable region of the heavy chain protein of 97B8E1 is from position 148 to position 198 in SEQ ID NO: 49 of the sequence listing;
the nucleotide sequence encoding the CDR3 region in the variable region of the heavy chain protein of 97B8E1 is from position 289 to position 330 in SEQ ID NO: 49 of the sequence listing;
the nucleotide sequence encoding the CDR1 region in the variable region of the light chain protein of 97B8E1 is from position 73 to position 105 in SEQ ID NO: 50 of the sequence listing;
the nucleotide sequence encoding the CDR2 region in the variable region of the light chain protein of 97B8E1 is from position 148 to position 174 in SEQ ID NO: 50 of the sequence listing;
the nucleotide sequence encoding the CDR3 region in the variable region of the light chain protein of 97B8E1 is from position 265 to position 291 in SEQ ID NO: 50 of the sequence listing.

### Example 4 Transformation and preparation of fully human antibody IgG

(I) Plasmid construction and preparation: purified CTLA-4 antibody from the culture supernatant of hybridoma cells had been obtained in Example 2, and according to the sequencing results of Example 7, the sequences of heavy chain variable region and light chain variable region of CTLA-4 antibody was clear. The heavy chain variable region sequence of the CTLA-4 antibody was recombined into an expression vector containing the signal peptide and the human heavy chain antibody IgG1 constant region (the expression vector was purchased from Invitrogen), and the light chain variable region sequence of the CTLA-4 antibody was recombined into an expression vector containing the signal peptide and the human antibody light chain kappa constant region. The recombinant plasmid was obtained and verified by sequencing (the sequencing method was the same as that in Example 7). Plasmids with a mass of more than 500µg were extracted using alkaline lysis kit (purchased from MACHEREY-NAGEL) to increase the purity, filtered through a 0.22µm filter membrane (purchased from Millopore) for transfection.
(II) Cell transfection: 293E cells (purchased from Invitrogen) were cultured in Freestyle 293 expression medium (purchased from Invitrogen). The shaker was set to 37°C, 130RPM, and 8% CO₂ (v/v) concentration.
   During transfection, Freestyle 293 expression medium was added with 10% (v/v) F68 (purchased from Invitrogen) to a final concentration of 0.1% (v/v), to obtain Freestyle 293 expression culture containing 0.1% (v/v) F68, that is, medium A.
   5mL of medium A was taken and mixed well with 200µg/mL PEI (purchased from Sigma), to obtain medium B. 5 mL of medium A was taken and mixed well with 100µg/mL of the recombinant plasmid obtained in step (I) to obtain medium C. 5 minutes later, medium B and medium C were combined and mixed, and the mixture was let stand for 15 minutes to obtain a mixture D. 10mL of mixture D was slowly added into 100mL of Freestyle 293 expression medium containing 293E cells, until the cell density of 293E was 1.5×10⁶/mL, and it was shaked while being added, to avoid excessive aggregation of PEI. And the mixture was placed in a shaker for culture. Peptone was added the next day to a final concentration of 0.5% (w/v). On days 5-7, the antibody titer of the culture medium was measured. On days 6-7, the supernatant was collected by centrifugation (3500RPM, 30 minutes) and filtered through a 0.22µm filter to obtain the filtered cell supernatant for purification.
(III) Antibody purification: For continuouly used endotoxin-free chromatography columns and Protein A stuffing (purchased from GE), 0.1M NaOH was used for washing for 30 minutes, or 5 column volumes of 0.5M NaOH was used for washing. For long-term unused column materials and chromatography columns, at least 1M NaOH was used for soaking for 1 hour, and non-endotoxic water was used for rinsing to neutrality, and the column material was washed with 10 column volumes of 1% (v/v) Triton×100. 5 column volumes of PBS (PBS phosphate buffer, pH 7.2) was used for equilibrate. The filtered cell supernatant obtained in step (II) was loaded on the column, and the flow-through liquid was collected if necessary. After the samples were loaded, the column was washed with 5 column volumes of PBS. Elution was performed with 5 column volumes of 0.1M pH3.0 Glycine-HCl, and the eluate was collected, and neutralized with 0.5 column volume of 1M Tris-HCl (1.5M NaCl) with pH 8.5. And fully human CTLA-4 antibody was obtained. All the above-mentioned solutions required a fresh configuration. After the fully human CTLA-4 antibodies harvested, they were dialyzed for 4 hours in 1×PBS to avoid endotoxin contamination. After dialysis, spectrophotometry or a kit was used to determine the concentration, and HPLC-SEC was used to determine the purity of the antibody, and an endotoxin detection kit (purchased from Lonza) was used to detect the content of antibody endotoxin.

### Example 5 Detection of the binding of fully human antibody to CTLA-4 expressing cells by Flow cytometry (FACS)

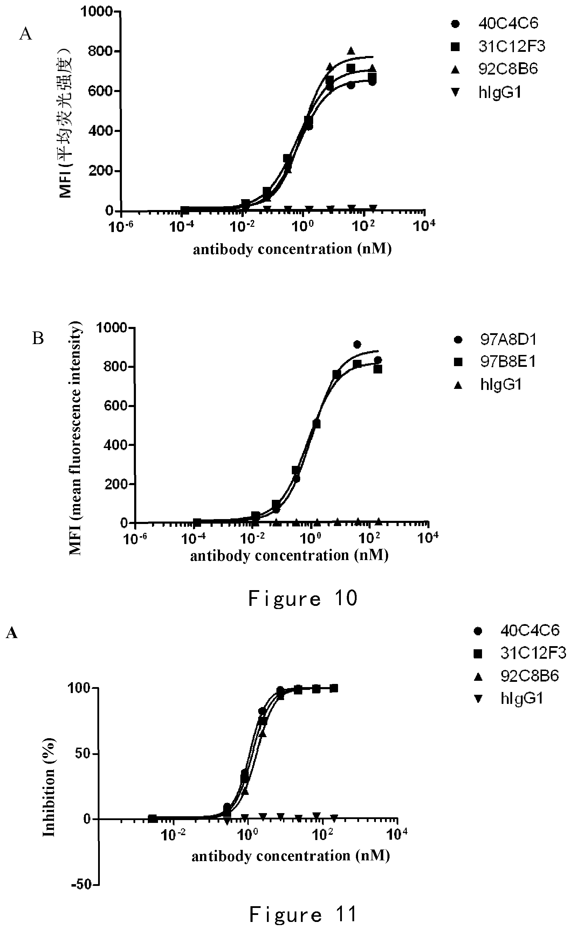

The binding activity of the obtained fully human CTLA-4 antibody to cells expressing CTLA-4 was identified, and the detection results are shown in Figure 9, Figure 10, Table 10 and Table 11, respectively.

**Table 10 Detection of the binding of antibodies to human CTLA-4 expressing cells by Flow cytometry (FACS)**

| Clone ID | Mean fluorescence intensity | | | | | | | | | |
|---|---|---|---|---|---|---|---|---|---|---|
| | Antibody Concentration (nM) | | | | | | | | | |
| | 200 | 40 | 8 | 1.6 | 0.32 | 0.064 | 0.0128 | 0.000128 | 200 | 40 |
| 97A8D1 | 1016 | 1017 | 919 | 580 | 196 | 66 | 22 | 3 | 1016 | 1017 |
| 92C8B6 | 1061 | 1035 | 827 | 509 | 203 | 67 | 23 | 3 | 1061 | 1035 |
| 31C12F3 | 998 | 941 | 756 | 547 | 260 | 96 | 34 | 3 | 998 | 941 |
| 40C4C6 | 963 | 967 | 795 | 553 | 233 | 74 | 24 | 3 | 963 | 967 |
| 97B8E1 | 886 | 902 | 727 | 491 | 229 | 85 | 31 | 3 | 886 | 902 |
| IgG control | 6 | 4 | 3 | 3 | 3 | 3 | 3 | 3 | 6 | 4 |

**Table 11 Detection of the binding of antibodies to monkey CTLA-4 expressing cells by Flow cytometry (FACS)**

| Clone ID | Mean fluorescence intensity | | | | | | | |
|---|---|---|---|---|---|---|---|---|
| | Antibody concentration (nM) | | | | | | | |
| | 200 | 40 | 8 | 1.6 | 0.32 | 0.064 | 0.0128 | 0.000128 |
| 97A8D1 | 831 | 910 | 754 | 513 | 225 | 67 | 23 | 2 |
| 92C8B6 | 711 | 800 | 722 | 465 | 208 | 68 | 22 | 2 |
| 31C12F3 | 668 | 712 | 654 | 455 | 261 | 98 | 34 | 2 |
| 40C4C6 | 643 | 626 | 619 | 421 | 219 | 69 | 23 | 2 |
| 97B8E1 | 784 | 810 | 757 | 504 | 270 | 97 | 36 | 2 |
| IgG control | 5 | 3 | 3 | 3 | 2 | 3 | 3 | 3 |

The results show that the fully human CTLA-4 antibody prepared and obtained after the transformation of fully human IgG can bind to human and monkey CTLA-4 with high activity, and the antibody activity was close.

### Example 6 Detection of the blocking effect of CTLA-4 antibodies on the binding of CTLA-4 protein to its receptor B7.1 or B7.2

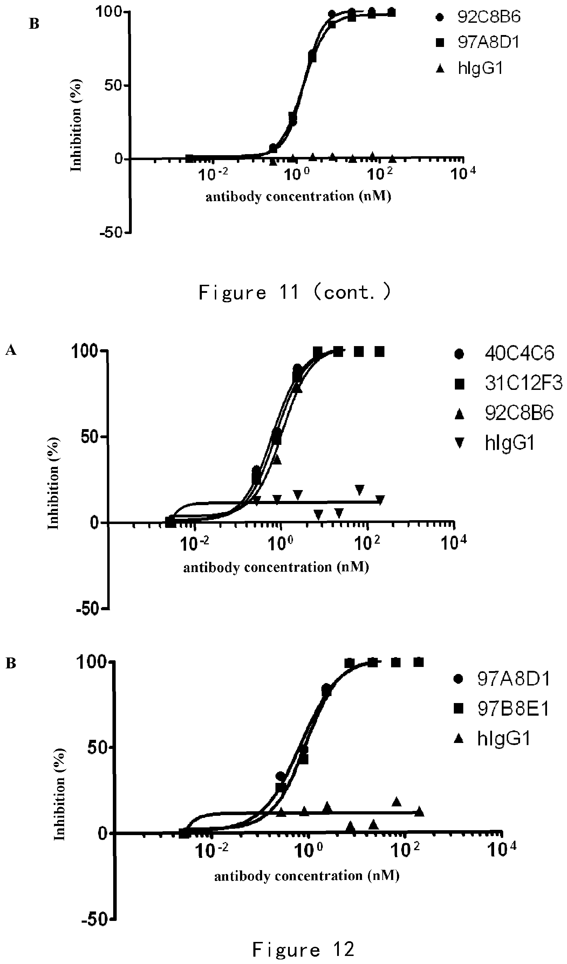

The blocking activity of the obtained fully human CTLA-4 antibody was identified, and the detection results are shown in Figure 11, Figure 12, Table 12, and Table 13, respectively.

**Table 12 The inhibition of the fully human CTLA-4 antibodies on the binding of CTLA-4 protein to its receptor B7.1**

| Clone ID | % Block | | | | | | | |
|---|---|---|---|---|---|---|---|---|
| | Antibody concentration (nM) | | | | | | | |
| | 200 | 40 | 8 | 1.6 | 0.32 | 0.064 | 0.0128 | 0.000128 |
| 40C4C6 | 99.4 | 99.4 | 99.2 | 98.1 | 82.1 | 35 | 9.3 | 0 |
| 31C12F3 | 99.3 | 99.1 | 98.5 | 95.9 | 74.5 | 30.4 | 5.1 | 0 |
| 92C8B6 | 99.3 | 98.9 | 98.1 | 93.7 | 65.1 | 21.4 | 5.3 | 0 |
| 92C8B6 | 99.5 | 99.4 | 99.3 | 97.7 | 71.2 | 24.4 | 7.2 | 0 |
| 97A8D1 | 98.4 | 97.2 | 95.2 | 90.6 | 67.8 | 28.7 | 6.1 | 0 |
| hIgG1 | -0.3 | 1.6 | -0.1 | 1.3 | 1 | 0.2 | -2 | 0 |

**Table 13 The inhibition of the fully human CTLA-4 antibodies on the binding of CTLA-4 protein to its receptor B7.2**

| Clone ID | % Block | | | | | | | |
|---|---|---|---|---|---|---|---|---|
| | Antibody concentration (nM) | | | | | | | |
| | 200 | 40 | 8 | 1.6 | 0.32 | 0.064 | 0.0128 | 0.000128 |
| 40C4C6 | 99.1 | 99.1 | 99.1 | 99.1 | 89.2 | 52.4 | 30 | 0 |
| 31C12F3 | 99.2 | 99.1 | 99.1 | 99 | 84.4 | 48.1 | 25.6 | 0 |
| 92C8B6 | 99.2 | 99.2 | 99.2 | 98.7 | 78.5 | 36.7 | 25 | 0 |
| hIgG1 | 12.4 | 18.3 | 4.9 | 4.1 | 15.5 | 12.9 | 12.3 | 0 |
| 97A8D1 | 99.2 | 99.2 | 99.2 | 99 | 84.1 | 48.4 | 33 | 0 |
| 97B8E1 | 99.1 | 99 | 99 | 98.7 | 82.3 | 42.8 | 26.3 | 0 |
| hIgG1 | 12.4 | 18.3 | 4.9 | 4.1 | 15.5 | 12.9 | 12.3 | 0 |

Results in Figure 11, Figure 12 and Table 12, Table 13 show that the fully human CTLA-4 antibody prepared after the transformation of fully human IgG can block the binding of CTLA-4 to B7.1 and B7.2, wherein the blocking activities of the tested antibodies were equivalent.

### Example 7 Detection of the effect of CTLA-4 antibody on lymphocyte activity in lymphocyte stimulation test

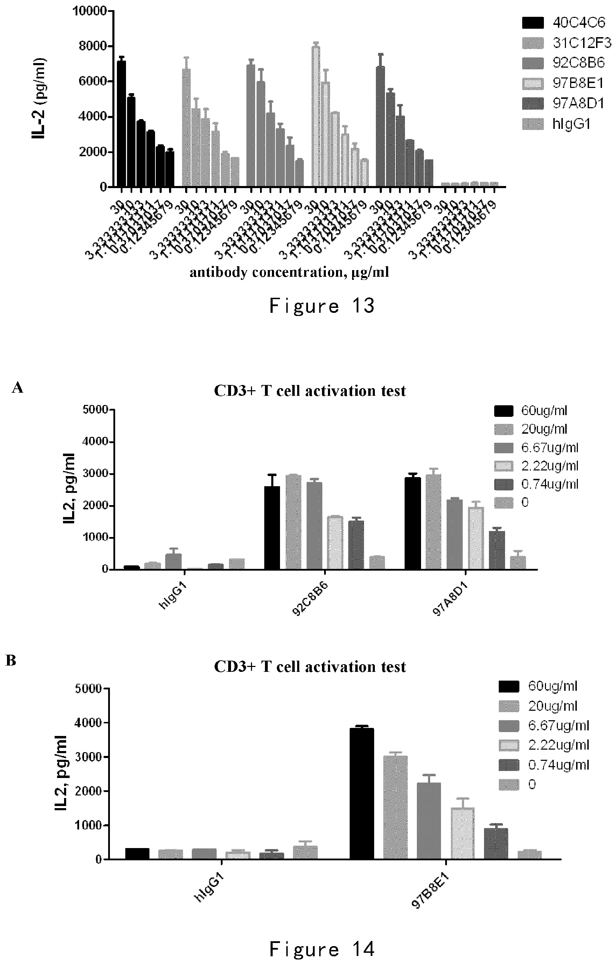

Experimental method was according to that of Example 2 (III). The test results are shown in Table 14 and Figure 13.

**Table 14 The effects of CTLA-4 fully human antibodies on IL-2 secretion in the SEB-dependent PBMC activation test**

| Clone ID | IL-2 secretion, pg/ml | | | | | | | | | | | |
|---|---|---|---|---|---|---|---|---|---|---|---|---|
| | 30ug/ml | | 10ug/ml | | 3.3ug/ml | | 1. 1ug/ml | | 0.37ug/ml | | 0.13ug/ml | |
| | N=1 | N=2 | N=1 | N=2 | N=1 | N=2 | N=1 | N=2 | N=1 | N=2 | N=1 | N=2 |
| 40C4C6 | 6839 | 7408 | 5267 | 4813 | 3773 | 3618 | 3199 | 3008 | 2369 | 2162 | 2135 | 1826 |
| 31C12F3 | 5924 | 7372 | 5021 | 3811 | 4422 | 3327 | 2647 | 3631 | 1765 | 2001 | 1656 | 1634 |
| 92C8B6 | 7240 | 6592 | 6674 | 5205 | 4856 | 3491 | 3606 | 2928 | 2811 | 1858 | 1567 | 1386 |
| 97B8E1 | 7653 | 8206 | 6649 | 5209 | 4240 | 4200 | 3455 | 2555 | 2481 | 1865 | 1572 | 1447 |
| 97A8D1 | 6088 | 7542 | 5075 | 5562 | 4664 | 3331 | 2660 | 2610 | 2114 | 1956 | 1516 | 1504 |
| hIgG1 | 210 | 210 | 196 | 190 | 208 | 219 | 166 | 274 | 220 | 254 | 257 | 221 |

It can be seen from Table 14 and Figure 13 that the fully humanized antibody can significantly stimulate IL-2 secretion in the SEB-dependent PBMC activation test, and this activity had a concentration gradient-dependent effect, indicating that the CTLA-4 antibody can reverse the inhibition of T cell activation by CTLA-4. And the activity levels of antibodies tested were equivalent. Wherein, the IgG control was human IgG1 (hIgG1), and the data in Table 14 is the IL-2 concentration.

### Example 8 Detection of the activation of T lymphocytes after the blocking of binding between B7.1 or B7.2 and CTLA-4 by CTLA-4 antibody in the PHA induced CD3+ T lymphocytes assay

The activation of T lymphocytes after the blocking of binding between B7.1 or B7.2 and CTLA-4 by CTLA-4 antibody was detected.

T cells constitutively express CD28, and PHA can activate T cells non-specifically, thereby inducing the membrane expression of CTLA-4. Raji cells constitutively express the ligands of CTLA-4/CD28, B7.1 and B7.2. When activated T cells are incubated with Raji cells, CTLA-4 and CD28 competitively bind to ligands. Since CTLA-4 has a higher affinity for ligands than CD28, when incubated with Raji cells, it shows a partial down-regulation of T cell activation. When the ligand binding ability of CTLA-4 is blocked, the down-regulation of T cell activation will be reversed, which is manifested as an increase in IL-2 secretion.

### (I) Activation of human CD3+ cells induced by PHA

PBMC cells were isolated from whole blood using reagent FICOLL PAQUE PLUS according to the instructions. The specific steps were as described in (5) in 4.4.2.

MagCellectTM Human CD3+ T Cell Isolation Kit was used to operate the obtained PBMC cells according to the instructions, thereby obtaining isolated and purified CD3+ cells. Human CD3+ T cells were inoculated into a 6-well plate and added with 10µg/mL PHA, treated for 72 hours to obtain CD3+ T cell capsules overexpressing CTLA-4.

### (II) Treatment of Raji Cells with mitomycin

Before used, Raji cells were treated with 10µg/mL mitomycin, at 37° C for 1.5 hours, and washed 3 times with PBS to remove residual mitomycin. Meanwhile, the purified CTLA-4 antibody diluted in equal volume ratio was prepared to obtain the sample solution to be tested.

### (III) Raji-mediated activation of T cell capsule

CD3+ T cell capsules obtained in the step① were inoculated into a 96-well cell culture plate at a density of 1×10⁵ cells/well, with an inoculation volume of 50 µL per well. And the plate was added with 50µL of the sample solution to be tested per well, and incubated together for 30 minutes, then added with the Raji cells of the step②, wherein the cells was spreaded as 100 µL 3 × 10⁴ cells/well on the 96 cell culture plate, and the plate was incubated in a 37° C, 5% CO₂ incubator for 4 days. The cell supernatant was collected for cytokine detection, and the percentage was the volume percentage.

### (IV) Detection of cytokine interleukin IL-2 in cell supernatant using enzyme-linked immunosorbent assay

Detection of cytokine interleukin IL-2 in cell supernatant obtained in step ③ was carried out using enzyme-linked immunosorbent assay. The R&D system related detection kit Quantikine ELISA human IL-2 (S2050) was used, and operated in accordance with the instructions. All test reagents except the tested antibodies were provided by the test kit.

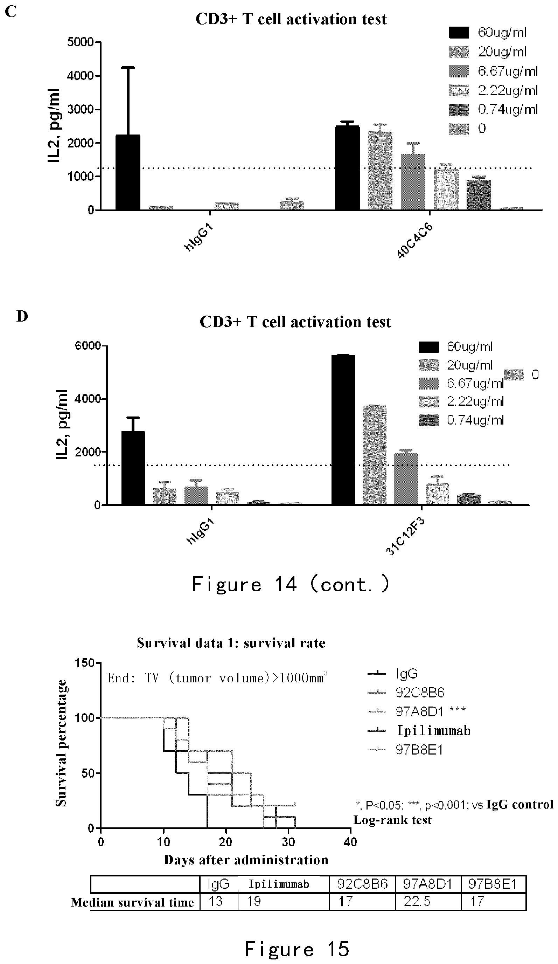

The effects of CTLA-4 fully human antibodies on IL-2 secretion in the PHA-induced T lymphatic sac cell activation test are shown in Figure 14. The results show that all the tested antibodies can stimulate T cells to secrete IL2, and the effect was dose-dependent with the antibody concentration. That is, the IL2 level increased as the antibody concentration increased, and 92C8B6 and 97A8D1 had the best activity.

### Example 9 Analysis and determination of anti-CTLA-4 antibody affinity

Anti-human Fc IgG was fixed on flow cell 1 and 2. HBS-EP+ (10mmol/L HEPES, 150mmol/L NaCl, 3mM EDTA, 0.05% P20, pH 7.4) was used as the running buffer, and anti-human Fc IgG was fixed using the fixing wizard template. The flow cells 1 and 2 connected in series with S CM5 sensor chip were activated with freshly mixed 50mmol/L NHS and 200mmol/L EDC. Anti-human Fc IgG was diluted to 20µg/mL with 10mmol/L NaAC (pH 4.5) and injected into activated flow cells 1 and 2. The remaining active coupling sites were blocked with 1mol/L ethanolamine.

The recombinant His-labeled hCTLA-4 ECD protein was diluted to 50nmol/L, and then serially diluted 4 times with HBS-EP+ buffer at a 2-fold ratio. The concentrations of His-labeled hCTLA-4 ECD proteins were 0nmol/L, 3.125nmol/L, 6.25nmol/L, 12.5nmol/L, 25nmol/L and 50nmol/L. HBS-EP+ was used as the running buffer for KD measurement. Each antibody was injected into the CM5 sensor flow cell 2 at a flow rate of 10 µL/min to achieve a response of 230RU. Then the prepared His-labeled hCTLA-4ECD protein was injected into flow cells 1 and 2 at a flow rate of 30 µL/min for 180 seconds. The buffer flow was maintained for 400 seconds for dissociation measurement (30µL/min). To remove the tested antibody from the surface, 10mmol/L glycine-HCl pH1.5 was injected for 20 seconds (30µL/min). Flow cell 1 was used as a reference flow cell. The above steps were repeated for each concentration of serially diluted His-labeled CTLA-4ECD protein. The KD value of each antibody was evaluated using Biacore T200 evaluation software 1.0, and a 1:1 binding model was used to fit the data. The results are shown in Table 15.

**Table 15 Analysis and determination of anti-CTLA-4 antibody affinity**

| Clone ID | K_{D} (nM) | kₐ (1/Ms) | k _{d}(l/s) |
|---|---|---|---|
| 97A8D1 | 2.15E-09 | 4.05E+05 | 8.70E-04 |
| 92C8B6 | 2.52E-09 | 7.44E+05 | 0.001873 |
| 31C12F3 | 2.62E-09 | 4.50E+05 | 0.00118 |
| 40C4C6 | 2.36E-09 | 7.20E+05 | 0.0017 |
| 97B8E1 | 3.50E-09 | 4.31E+05 | 0.001506 |

The results show that the KD values of the tested antibodies were all at the nanomolar level and were comparable to the tool antibodies, indicating that these antibodies had a good affinity to human CTLA-4 ECD. Wherein, the 97A8D1 antibody had the best affinity to human CTLA-4 ECD, which is suitable as a candidate antibody for *in vivo* activity confirmation.

### Example 10 Evaluation of anti-tumor activity of anti-CTLA-4 antibody in mice

The MC38 syngeneic mouse model, C57BL/6 mice knocked-in with human CTLA-4 gene were used to evaluate the anti-tumor activity of antibodies in mice. The experiment was designed as following. 50 human CTLA-4 knock-in C57BL/6 mice were selected and divided into 6 groups, each with 10 mice. Ipilimumab and the isotype antibody hIgG1 were used as controls. The samples were 92C8B6, 97A8D1 and 97B8E1. The route of administration was intraperitoneal injection with a dosage of 10mg/kg, on days 0, 3, 6, and 10. And the mice were sacrificed on day 24. The tumor volume, mouse body weight, tumor weight and mouse survival rate were measured. The experimental results are shown in Figure 15.

The results of Figure 15 show that the antibodies of the present invention (including 92C8B6, 97A8D1 and 97B8E1) can significantly inhibit tumor growth, and the inhibitory effects were significantly better than that of the control antibody Ipilimumab (for example, the median survival time of 97A8D1 was 22.5 days, which is better than that of Ipilimumab, 19 days).

### Example 11 Preparation and characterization of defucosylated anti-CTLA-4 antibody

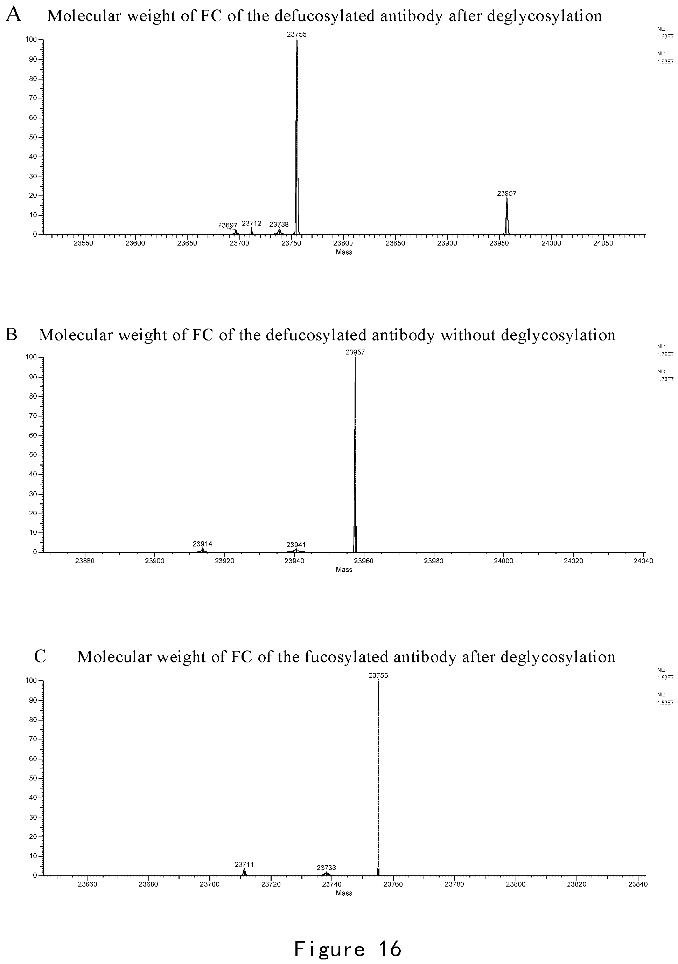

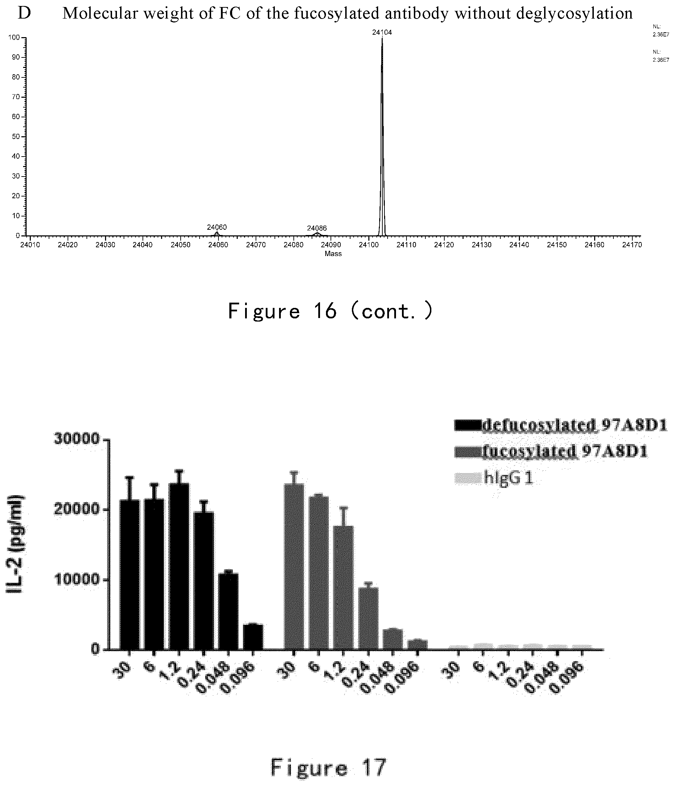

In this example, a fully human anti-CTLA-4 antibody 97A8D1 was expressed in a cell line lacking fucosyltransferase, which allowed the cell line to produce defucosylated protein. LC/MS technology was used to analyze the molecular weights of defucosylated antibody 97A8D1 and fucosylated antibody 97A8D1 after digestion with Ide Z and EndoS, to determine whether the antibody Fc terminal contained fucose. The analysis results are shown in Figure 16.

The results in Figure 16 show that after enzymatic treatment, the molecular weight of Fc after deglycosylation of defucosylated antibody 97A8D1 was 23755 Da (Figure 16A), while the molecular weight of Fc without deglycosylation was 23957 Da (Figure 16B). The molecular weight difference between the two was 202 Da (GlcNAc). After enzymatic treatment, the molecular weight of Fc after deglycosylation of fucosylated antibody 97A8D1 was 23755Da (Figure 16C), and the molecular weight of Fc without deglycosylation was 24104Da (Figure 16D). The difference in molecular weight between the two was 349Da (Fuc+GlcNAc). Since the molecular weight of the Fc of the defucosylated antibody (23957Da) was 147Da less than that of the Fc of the fucosylated antibody (24104Da), it was speculated at the molecular weight level that the defucosylated antibody did not contain fucose and fucosylated antibodies contained fucose.

### Example 12 Detection of the effect of defucosylated anti-CTLA-4 antibody on lymphocyte activity in lymphocyte stimulation test

Experimental method was according to that of Example 2 (III). The test results are shown in Table 18 and Figure 17.

**Table 18 The effects of defucosylated and fucosylated anti-CTLA-4 antibodies on IL-2 secretion in the SEB-dependent PBMC activation test**

| Clone ID | IL-2 secretion, pg/ml | | | | | | | | | | | |
|---|---|---|---|---|---|---|---|---|---|---|---|---|
| | 30ug/ml | | 6ug/ml | | 1.2ug/ml | | 0.24ug/ml | | 0.048ug/ml | | 0.0096ug/ml | |
| | N=1 | N=2 | N=1 | N=2 | N=1 | N=2 | N=1 | N=2 | N=1 | N=2 | N=1 | N=2 |
| Defucosylated 97A8D1 | 17792 | 24639 | 19119 | 23609 | 25546 | 21592 | 17778 | 21179 | 10239 | 11260 | 3230 | 3632 |
| Fucosylated 97A8D1 | 21635 | 25325 | 21230 | 22142 | 14892 | 20251 | 7824 | 9596 | 2574 | 2988 | 1436 | 1024 |
| hIgG1 | 432 | 480 | 609 | 871 | 507 | 634 | 760 | 634 | 515 | 615 | 581 | 548 |

From Table 18 and Figure 17, it can be seen that both defucosylated and fucosylated anti-CTLA-4 antibodies can reverse the inhibitory effect of CTLA-4 on T cell activation, but the activity of defucosylated anti-CTLA-4 antibody was better than that of the fucosylated anti-CTLA-4 antibody.

### Discussion

(1) Antibodies can be obtained by immunizing wild-type mice, but mouse antibodies need to be humanized to obtain humanized antibodies. The disadvantage is that the modified antibody may be more immunogenic and the structure of the antibody may be changed resulting in loss of activity or poor manufacturability.
(2) Fully human antibodies can be obtained by immunizing fully human transgenic mice, but the number or affinity of the obtained antibodies will be poor.
(3) Antibody expression and activity screening can be carried out by constructing immunized mouse antibody library with phage display technology, but the random recombination of antibody heavy and light chains will result in poor production of the formed antibodies.
(4) The antibody can be expressed and screened by phage display technology by constructing a human antibody library. But the affinity of the obtained antibody will be poor because it has not been immunized.
(5) The immunogen can be polypeptides, proteins, other types of cells and genes, but there will be problems such as incorrect conformation, low expression, and poor immunogenicity.

All publications mentioned herein are incorporated by reference as if each individual document was cited as a reference in the present application. It should be understood that, after reading the above teachings of the present invention, those skilled in the art can make various modifications and changes. These equivalent forms are also within the scope defined by the claims appended hereto.

The sequence information involved in the present invention is as follows:
The amino acid sequences of the CTLA-4 antibody correspond to the amino acid sequence listed, wherein CDR1, CDR2, CDR3, CDR1', CDR2', and CDR3' are underlined respectively:

SEQ ID No.1 >97A8D1_VH (mAb49)
QVQLVESGGG VVQPGRSLRL SCVASGFTFN NYGMNWVRQA PGKGLEWVAV IWYGGRNKFY ADSVKGRFTI SRDNSKNTLY
LEMNSLRAED TAVYYCARAD WGGWFDPWGQ GTLVTVSS

SEQ ID No.5 >97A8D1_VL (mAb49)
EIVLTQSPGT LSLSPGERAT LSCRASQSVS STYLAWYQQK PGQAPRLLIH GAFSRATGIP DRFSGSGSGP DFTLTISRLE
PEDFAVYYCQ QYGTSPFTFG PGTKVDIK

SEQ ID No.9>92C8B6_VH (mAb42)
QVQLVESGGG VVQPGRSLRL SCAASGFTFS NFGMHWVRQA PGKGLEWVAV IWYDGSNKYY ADSVKGRFTI SRDNSKNTLF
LQMNGLRAED TAVYYCAKGG ILMAGTLDYW GQGTLVTVSS

SEQ ID No.13>92C8B6_VL (mAb42)
DIQMTQSPSS LSASVGDRVT ITCRASQSVS TYLNWYQQKP GKAPKLLIYA ASSFQSGVPS RFSGSGSGTD FTLTISSLQP
EDFATYFCQQ SYSTPFTFGP GTKVDIK

SEQ ID No.17>31C12F3_VH (mAb010)
QVQLVESGGG VVQPGRSLIL SCAASGFTFS SYGMHWVRQA PGKGLEWVAV VWYDGSNKYY ADSVKGRFTI SRDNSKNTLY
LQMSSLRAED TAVYYCARGG IVVPGLFEYW GQGSLVTVSS

SEQ ID No.21>31C12F3_VL (mAb010)
DIQMTQSPSS LSASVGDRVA ITCRTSQSIS NLLNWYQQKP GKAPELLIYA PSSFQSGVPS RFRGSGSGTD FTLTISSLQP
EDFATYYCQQ SYSTPFTFGP GTKVDIK

SEQ ID No.25>40C4C6_VH (mAb09)
QVQLVESGGG VVQPGRSLRL SCAASGFTFS SYGMHWVRQA PGKGLEWVAV IWYDGRNKYY ADSVKGRFTI SRDNSKNTLY
LQMNSLRAED TAVYYCARAS GSYSYYFDYW GQGTLVTVSS

SEQ ID No.29>40C4C6_VL (mAb09)
DIQMTQSPSS LSASVGGRVT ITCRASQSIS SFLNWYQHEL GKAPKLLIYG ASSLQSGVPS RFSGSGSGTD FTLTISSLQP
EDFATYYCQQ TYSTPFTFGP GTKVDIK

SEQ ID No.33>97B8E1_VH (mAb50)
QVQLVESGGG VVQPGRSLRL SCAASGFTFS SYVMHWVRQA PGKGLEWVAV IWSDGRNKYY TDSVKGRFTI SRDNSKNTLY
LQMSSLRAED TAVYYCARSG IALAGNAFDI WGQGTMVTVS S

SEQ ID No.37>97B8E1_VL (mAb50)
DIQMTQSPSS LSASVGDRVT ITCRASQSIS SYLNWYQQKP GKAPKLLIYA ASSLQSGVPS RFSGSGSGTD FTLTISSLQP
EDFATYYCQQ SYSTPFTFGP GTKVDIK

The nucleotide sequences encoding the corresponding CTLA-4 antibody amino acid sequences are listed, wherein the nucleotide sequences encoding the CDR1, CDR2, CDR3, CDR1' CDR2', and CDR3' are underlined respectively:

SEQ ID No.41_97A8D1_VH (mAb49)
CAGGTGCAGCTGGTGGAGTCTGGGGGAGGCGTGGTCCAGCCTGGGAGGTCCCTGAGACTCTCCTGTGTAGCGTCTGGATTCACCTTCAATAA
CTATGGCATGAACTGGGTCCGCCAGGCTCCAGGCAAGGGGCTGGAGTGGGTGGCAGTTATTTGGTATGGTGGACGTAATAAATTCTATGCAG
ACTCCGTGAAGGGCCGATTCACCATTTCCAGAGACAATTCCAAGAACACGCTGTATCTGGAAATGAACAGCCTGAGAGCCGAGGACACGGCT
GTGTATTACTGTGCGAGAGCTGACTGGGGAGGGTGGTTCGACCCCTGGGGCCAGGGAACCCTGGTCACCGTCTCCTCA

SEQ ID No.42_97A8D1_VL (mAb49)
GAAATTGTGTTGACGCAGTCTCCAGGCACCCTGTCTTTGTCTCCAGGGGAAAGAGCCACCCTCTCCTGCAGGGCCAGTCAGAGTGTTAGCAG
CACCTACTTAGCCTGGTACCAGCAGAAACCTGGCCAGGCTCCCAGGCTCCTCATCCATGGTGCATTCAGCAGGGCCACTGGCATCCCAGACA
GGTTCAGTGGCAGTGGGTCTGGGCCAGACTTCACTCTCACCATCAGCAGACTGGAGCCTGAAGATTTTGCAGTTTATTACTGTCAGCAGTAT
GGTACCTCACCATTCACTTTCGGCCCTGGGACCAAAGTGGATATCAAA

SEQ ID No.43_92C8B6_VH (mAb42)
CAGGTGCAGCTGGTGGAGTCTGGGGGAGGCGTGGTCCAGCCTGGGAGGTCCCTGAGACTCTCCTGTGCAGCGTCTGGATTCACCTTCAGTAA
CTTTGGCATGCACTGGGTCCGCCAGGCTCCAGGCAAGGGGCTGGAGTGGGTGGCAGTTATATGGTATGATGGAAGTAATAAATACTATGCAG
ACTCCGTGAAGGGCCGATTCACCATCTCCAGAGACAATTCCAAGAACACGCTGTTTCTGCAAATGAACGGCCTGAGAGCCGAGGACACGGCT
GTGTATTACTGTGCGAAAGGGGGTATATTAATGGCTGGTACGCTGGACTACTGGGGCCAGGGAACCCTGGTCACCGTCTCCTCA

SEQ ID No.44_92C8B6_VL (mAb42)
GACATCCAGATGACCCAGTCTCCATCCTCCCTGTCTGCATCTGTAGGAGACAGAGTCACCATCACTTGCCGGGCAAGTCAGAGCGTTAGCAC
CTATTTAAATTGGTATCAGCAGAAACCAGGGAAAGCCCCTAAGCTCCTGATCTATGCTGCATCCAGTTTCCAAAGTGGGGTCCCATCAAGGT
TCAGTGGCAGTGGATCTGGGACAGATTTCACTCTCACCATCAGCAGTCTGCAACCTGAAGATTTTGCAACTTACTTCTGTCAACAGAGTTAC
AGTACCCCATTCACTTTCGGCCCTGGGACCAAAGTGGATATCAAA

SEQ ID No.45_31C12F3_VH (mAb010)
CAGGTGCAGCTGGTGGAGTCTGGGGGAGGCGTGGTCCAGCCTGGGAGGTCCCTGATACTCTCCTGTGCAGCGTCTGGTTTCACCTTCAGTAG
CTATGGCATGCACTGGGTCCGCCAGGCTCCAGGCAAGGGGCTGGAGTGGGTGGCAGTTGTTTGGTATGATGGAAGTAATAAATACTATGCAG
ACTCCGTGAAGGGCCGATTCACCATCTCCAGAGACAATTCCAAGAACACGCTGTATCTGCAAATGAGCAGCCTGAGAGCCGAGGACACGGCT
GTGTATTACTGTGCGAGAGGGGGTATAGTAGTGCCTGGCCTCTTTGAGTACTGGGGCCAGGGATCCCTGGTCACCGTCTCCTCA

SEQ ID No.46_31C12F3_VL (mAb010)
GACATCCAGATGACCCAGTCTCCATCCTCCCTGTCTGCATCTGTAGGAGACAGAGTCGCCATCACTTGCCGGACAAGTCAGAGCATTAGTAA
CTTGTTAAATTGGTATCAGCAGAAACCAGGGAAAGCCCCTGAGCTCCTGATCTATGCTCCATCCAGTTTCCAAAGTGGGGTCCCATCAAGGT
TCAGGGGCAGTGGATCTGGGACAGATTTCACTCTCACCATCAGTAGTCTGCAACCTGAAGATTTTGCAACTTACTACTGTCAACAGAGTTAC
AGTACCCCATTCACTTTCGGCCCTGGGACCAAAGTGGATATCAAA

SEQ ID No.47_40C4C6_VH (mAb09)
CAGGTGCAGTTGGTGGAGTCTGGGGGAGGCGTGGTCCAGCCTGGGAGGTCCCTGAGACTCTCCTGTGCAGCGTCTGGATTCACCTTCAGTAG
CTATGGCATGCACTGGGTCCGCCAGGCTCCAGGCAAGGGGCTGGAGTGGGTGGCAGTTATATGGTATGATGGAAGAAATAAATATTATGCAG
ACTCCGTGAAGGGCCGATTCACCATCTCCAGAGACAATTCCAAGAACACGCTGTATCTGCAAATGAACAGCCTGAGAGCCGAGGACACGGCT
GTATATTACTGTGCGAGGGCTTCGGGGAGTTATTCGTACTACTTTGACTACTGGGGCCAGGGAACCCTGGTCACCGTCTCCTCA

SEQ ID No.48_40C4C6_VL (mAb09)
GACATCCAGATGACCCAGTCTCCATCCTCCCTGTCTGCATCTGTAGGAGGCAGAGTCACCATCACTTGCCGGGCAAGTCAGAGCATTAGCAG
TTTTTTAAATTGGTATCAACATGAACTAGGGAAAGCCCCTAAACTCCTGATCTATGGTGCATCCAGTTTGCAAAGTGGGGTCCCATCA
AGGTTCAGTGGCAGTGGATCTGGGACAGATTTCACTCTCACCATCAGCAGTCTGCAACCTGAAGATTTTGCAACTTACTACTGTCAACAGAC
TTACAGTACCCCATTCACTTTCGGCCCTGGGACCAAAGTGGATATCAAA

SEQ ID No.49_97B8E1_VH (mAb50)

CAAGTGCAGCTGGTGGAGTCTGGGGGAGGCGTGGTCCAGCCTGGGAGGTCCCTGAGACTCTCCTGTGCAGCGTCTGGATTCACCTTCAGTAG
TTATGTCATGCACTGGGTCCGCCAGGCTCCAGGCAAGGGGCTGGAGTGGGTGGCAGTTATATGGTCTGATGGAAGAAATAAATACTATACAG
ACTCCGTGAAGGGCCGATTCACCATCTCCAGAGACAATTCCAAGAACACGCTGTATCTGCAAATGAGCAGCCTGAGAGCCGAGGACACGGCT
GTGTATTACTGTGCGCGGTCGGGTATAGCGCTGGCTGGTAACGCTTTTGATATCTGGGGCCAAGGGACAATGGTCACCGTCTCTTCA

SEQ ID No.50_97B8E1_VL (mAb50)
GACATCCAGATGACCCAGTCTCCATCCTCCCTGTCTGCATCTGTAGGAGACAGAGTCACCATCACTTGCCGGGCAAGTCAGAGCATTAGCAG
TTATTTAAATTGGTATCAGCAGAAACCAGGGAAAGCCCCTAAGCTCCTGATCTATGCTGCATCCAGTTTGCAAAGTGGGGTCCCATCAAGGT
TCAGTGGCAGTGGATCTGGGACAGATTTCACTCTCACCATCAGCAGTCTGCAACCTGAAGATTTTGCAACTTACTACTGTCAACAGAGTTAC
AGTACCCCATTCACTTTCGGCCCTGGGACCAAAGTGGATATCAAA

**Table 16 CDR region sequences of CTLA-4 antibody**

| AA | | VH | | | VL | | |
|---|---|---|---|---|---|---|---|
| mAb# | clone# | CDR1 | CDR2 | CDR3 | CDR1' | CDR2' | CDR3' |
| mAb049 | 97A8D1 | nnygmn | viwyggrnkfyadsvkg | aradwggwfdp | asqsvsstylaw | gafsratgi | qqygtspft |
| mAb042 | 92C8B6 | snfgmh | viwydgsnkyyadsvkg | akggilmagtldy | asqsvstylnw | aassfqsgv | qqsystpft |
| mAb010 | 31C12F3 | ssygmh | vvwydgsnkyyadsvkg | arggivvpglfey | tsqsis--nllnw | apssfqsgv | qqsystpft |
| mAb009 | 40C4C6 | ssygmh | viwydgrnkyyadsvkg | arasgsysyyfdy | asqsis--sflnw | gasslqsgv | qqtystpft |
| mAb050 | 97B8E1 | ssyvmh | viwsdgrnkyytdsvkg | arsgialagnafdi | asqsissylnw | aasslqsgv | qqsystpft |

**Table 17 The sequence numbers of the CDR regions of the CTLA-4 antibody are as follows:**

| AA | | VH | | | VL | | |
|---|---|---|---|---|---|---|---|
| mAb# | Clone# | CDR1 SEQ ID No. | CDR2 SEQ ID No. | CDR3 SEQ ID No. | CDR1' SEQ ID No. | CDR2' SEQ ID No. | CDR3' SEQ ID No. |
| mAb049 | 97A8D1 | 2 | 3 | 4 | 6 | 7 | 8 |
| mAb042 | 92C8B6 | 10 | 11 | 12 | 14 | 15 | 16 |
| mAb010 | 31C12F3 | 18 | 19 | 20 | 22 | 23 | 24 |
| mAb009 | 40C4C6 | 26 | 27 | 28 | 30 | 31 | 32 |
| mAb050 | 97B8E1 | 34 | 35 | 36 | 38 | 39 | 40 |

### References:

[4] Ren Jun, Huang Hongyan, Current status and trend of tumor immunotherapy targeted at immune checkpoints [J]. Chinese Cancer Clinic, 2014, 41(7):415-419

## Claims

1. A heavy chain variable region of an antibody, wherein the heavy chain variable region comprises the following three complementary determining regions or CDRs:
CDR1 shown in SEQ ID NO: 8n+2,
CDR2 shown in SEQ ID NO: 8n+3, and
CDR3 shown in SEQ ID NO: 8n+4;
wherein, each n is independently 0, 1, 2, 3 or 4;
wherein, any one of the above amino acid sequences also includes a derivative sequence that is optionally with at least one amino acid added, deleted, modified, and/or substituted, and is capable of retaining the binding affinity to CTLA-4.

2. A heavy chain of an antibody, wherein the heavy chain comprises the heavy chain variable region of claim 1.

3. A light chain variable region of an antibody, wherein the light chain variable region comprises the following three complementary determining regions or CDRs:
CDR1' shown in SEQ ID NO: 8n+6,
CDR2' shown in SEQ ID NO: 8n+7, and
CDR3' shown in SEQ ID NO: 8n+8;
wherein, each n is independently 0, 1, 2, 3 or 4;
wherein, any one of the above amino acid sequences also includes a derivative sequence that is optionally with at least one amino acid added, deleted, modified, and/or substituted, and is capable of retaining the binding affinity to CTLA-4.

4. A light chain of an antibody, wherein the light chain comprises the light chain variable region of claim 3.

5. An antibody, wherein the antibody comprises:
(1) the heavy chain variable region of claim 1; and/or
(2) the light chain variable region of claim 3;
or the antibody comprises: the heavy chain of claim 2; and/or the light chain of claim 4,
wherein, any one of the above amino acid sequences also includes a derivative sequence that is optionally with at least one amino acid added, deleted, modified, and/or substituted, and is capable of retaining the binding affinity to CTLA-4.

6. The antibody of claim 5, wherein the antibody comprises the heavy chain variable region of claim 1 and the light chain variable region of claim 3;
wherein, the heavy chain variable region comprises the following three complementary determining regions or CDRs:
CDR1 shown in SEQ ID NO: 2,
CDR2 shown in SEQ ID NO: 3, and
CDR3 shown in SEQ ID NO: 4;
wherein, the light chain variable region comprises the following three complementary determining regions or CDRs:
CDR1' shown in SEQ ID NO: 6,
CDR2' shown in SEQ ID NO: 7, and
CDR3' shown in SEQ ID NO: 8;
wherein, any one of the above amino acid sequences also includes a derivative sequence that is optionally with at least one amino acid added, deleted, modified, and/or substituted, and is capable of retaining the binding affinity to CTLA-4.

7. The antibody of claim 5, wherein the heavy chain variable region of the antibody has the amino acid sequence shown in SEQ ID NO: 8n+1; and/or the light chain variable region of the antibody has the amino acid sequence shown in SEQ ID NO: 8n+5; wherein, each n is independently 0, 1, 2, 3 or 4.

8. The antibody of claim 6, wherein the heavy chain variable region of the antibody has the amino acid sequence shown in SEQ ID NO: 1, and the light chain variable region of the antibody has the amino acid sequence shown in SEQ ID NO: 5.

9. A recombinant protein, wherein the recombinant protein comprises:
(i) the heavy chain variable region of claim 1, the heavy chain of claim 2, the light chain variable region of claim 3, the light chain of claim 4, or the antibody of any one of claims 5-8; and
(ii) an optional tag sequence to assist expression and/or purification.

10. A polynucleotide, wherein the polynucleotide encodes a polypeptide selected from group consisting of:
(1) the heavy chain variable region of claim 1, the heavy chain of claim 2, the light chain variable region of claim 3, the light chain of claim 4, or any one of the antibodies of claims 5-8; and
(2) the recombinant protein of claim 9.

11. The polynucleotide of claim 10, wherein the polynucleotide encoding the heavy chain variable region is shown in SEQ ID NO: 41, 43, 45, 47 or 49; and/or,
the polynucleotide encoding the light chain variable region is shown in 42, 44, 46, 48 or 50.

12. The polynucleotide of claim 11, wherein the polynucleotide encoding the heavy chain variable region is shown in SEQ ID NO: 41; and the polynucleotide encoding the light chain variable region is shown in 42.

13. A vector, wherein the vector comprises the polynucleotide according to any one of claims 10-12.

14. A genetically engineered host cell, wherein the host cell contains the vector of claim 13 or the genome thereof is integrated with the polynucleotide of any one of claims 10-12.

15. A pharmaceutical composition, wherein the pharmaceutical composition comprises:
(i) an active ingredient, wherein the active ingredient is selected from the group consisting of: the heavy chain variable region of claim 1, the heavy chain of claim 2, the light chain variable region of claim 3, the light chain of claim 4, and the antibody of any one of claims 5-8, the recombinant protein of claim 9, and combinations thereof; and
(ii) a pharmaceutically acceptable carrier.

16. Use of an active ingredient, wherein the active ingredient is selected from the group consisting of: the heavy chain variable region of claim 1, the heavy chain of claim 2, the light chain variable region of claim 3, the light chain of claim 4, and the antibody of any one of claims 5-8, the recombinant protein of claim 9, and combinations thereof, wherein the active ingredient is used for preparation of a medicine for preventing and/or treating CTLA-4 related tumors and/or viral infection related diseases.

17. A composition for detecting CTLA-4 protein in a sample *in vitro,* which comprises the antibody of any one of claims 5-8, and the recombinant protein of claim 9, or a combination thereof, used as an active ingredient.


Sequence listing

<110>  SHANGHAI PHARMAEXPLORER CO., LTD.
       PHARMAEXPLORER LIMITED

<120>  Antibody targeting CTLA-4, preparation method therefor and use thereof

<130>  P2020-1728

<150>  CN201810207977.9
<151>  2018-03-14

<160>  50

<170>  PatentIn version 3.5

<210>  1
<211>  118
<212>  PRT
<213>  Artificial Sequence

<220>
<223>  97A8D1_VH

<400>  1

Gln Val Gln Leu Val Glu Ser Gly Gly Gly Val Val Gln Pro Gly Arg
1               5                   10                  15

Ser Leu Arg Leu Ser Cys Val Ala Ser Gly Phe Thr Phe Asn Asn Tyr
            20                  25                  30

Gly Met Asn Trp Val Arg Gln Ala Pro Gly Lys Gly Leu Glu Trp Val
        35                  40                  45

Ala Val Ile Trp Tyr Gly Gly Arg Asn Lys Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Glu Met Asn Ser Leu Arg Ala Glu Asp Thr Ala Val Tyr Tyr Cys
                85                  90                  95

Ala Arg Ala Asp Trp Gly Gly Trp Phe Asp Pro Trp Gly Gln Gly Thr
            100                 105                 110

Leu Val Thr Val Ser Ser
        115

<210>  2
<211>  6
<212>  PRT
<213>  Artificial Sequence

<220>
<223>  VH-CDR

<400>  2

Asn Asn Tyr Gly Met Asn
1               5


<210>  3
<211>  17
<212>  PRT
<213>  Artificial Sequence

<220>
<223>  VH-CDR

<400>  3

Val Ile Trp Tyr Gly Gly Arg Asn Lys Phe Tyr Ala Asp Ser Val Lys
1               5                   10                  15


Gly


<210>  4
<211>  11
<212>  PRT
<213>  Artificial Sequence

<220>
<223>  VH-CDR

<400>  4

Ala Arg Ala Asp Trp Gly Gly Trp Phe Asp Pro
1               5                   10


<210>  5
<211>  108
<212>  PRT
<213>  Artificial Sequence

<220>
<223>  97A8D1_VL

<400>  5

Glu Ile Val Leu Thr Gln Ser Pro Gly Thr Leu Ser Leu Ser Pro Gly
1               5                   10                  15


Glu Arg Ala Thr Leu Ser Cys Arg Ala Ser Gln Ser Val Ser Ser Thr
            20                  25                  30


Tyr Leu Ala Trp Tyr Gln Gln Lys Pro Gly Gln Ala Pro Arg Leu Leu
        35                  40                  45

Ile His Gly Ala Phe Ser Arg Ala Thr Gly Ile Pro Asp Arg Phe Ser
    50                  55                  60

Gly Ser Gly Ser Gly Pro Asp Phe Thr Leu Thr Ile Ser Arg Leu Glu
65                  70                  75                  80

Pro Glu Asp Phe Ala Val Tyr Tyr Cys Gln Gln Tyr Gly Thr Ser Pro
                85                  90                  95

Phe Thr Phe Gly Pro Gly Thr Lys Val Asp Ile Lys
            100                 105

<210>  6
<211>  12
<212>  PRT
<213>  Artificial Sequence

<220>
<223>  VL-CDR

<400>  6

Ala Ser Gln Ser Val Ser Ser Thr Tyr Leu Ala Trp
1               5                   10

<210>  7
<211>  9
<212>  PRT
<213>  Artificial Sequence

<220>
<223>  VL-CDR

<400>  7

Gly Ala Phe Ser Arg Ala Thr Gly Ile
1               5

<210>  8
<211>  9
<212>  PRT
<213>  Artificial Sequence

<220>
<223>  VL-CDR

<400>  8

Gln Gln Tyr Gly Thr Ser Pro Phe Thr
1               5

<210>  9
<211>  120
<212>  PRT
<213>  Artificial Sequence

<220>
<223>  92C8B6_VH

<400>  9

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asn Phe
            20                  25                  30

Gly Met His Trp Val Arg Gln Ala Pro Gly Lys Gly Leu Glu Trp Val
        35                  40                  45

Ala Val Ile Trp Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Phe
65                  70                  75                  80

Leu Gln Met Asn Gly Leu Arg Ala Glu Asp Thr Ala Val Tyr Tyr Cys
                85                  90                  95

Ala Lys Gly Gly Ile Leu Met Ala Gly Thr Leu Asp Tyr Trp Gly Gln
            100                 105                 110

Gly Thr Leu Val Thr Val Ser Ser
        115                 120


<210>  10
<211>  6
<212>  PRT
<213>  Artificial Sequence

<220>
<223>  VH-CDR

<400>  10

Ser Asn Phe Gly Met His
1               5


<210>  11
<211>  17
<212>  PRT
<213>  Artificial Sequence

<220>
<223>  VH-CDR

<400>  11

Val Ile Trp Tyr Asp Gly Ser Asn Lys Tyr Tyr Ala Asp Ser Val Lys
1               5                   10                  15

Gly

<210> 12
<211> 13
<212> PRT
<213> Artificial Sequence

<220>
<223> VH-CDR

<400> 12

Ala Lys Gly Gly Ile Leu Met Ala Gly Thr Leu Asp Tyr
1               5                   10

<210> 13
<211> 107
<212> PRT
<213> Artificial Sequence

<220>
<223> 92C8B6_VL

<400> 13

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Ser Thr Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Phe Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Phe Cys Gln Gln Ser Tyr Ser Thr Pro Phe
                85                  90                  95

Thr Phe Gly Pro Gly Thr Lys Val Asp Ile Lys
            100                 105

<210> 14
<211> 11
<212> PRT
<213> Artificial Sequence

<220>
<223>  VL-CDR

<400>  14

Ala Ser Gln Ser Val Ser Thr Tyr Leu Asn Trp
1               5                   10


<210>  15
<211>  9
<212>  PRT
<213>  Artificial Sequence

<220>
<223>  VL-CDR

<400>  15

Ala Ala Ser Ser Phe Gln Ser Gly Val
1               5


<210>  16
<211>  9
<212>  PRT
<213>  Artificial Sequence

<220>
<223>  VL-CDR

<400>  16

Gln Gln Ser Tyr Ser Thr Pro Phe Thr
1               5


<210>  17
<211>  120
<212>  PRT
<213>  Artificial Sequence

<220>
<223>  31C12F3_VH

<400>  17

Gln Val Gln Leu Val Glu Ser Gly Gly Gly Val Val Gln Pro Gly Arg
1               5                   10                  15


Ser Leu Ile Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
        35                  40                  45


Ala Val Val Trp Tyr Asp Gly Ser Asn Lys Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Ser Ser Leu Arg Ala Glu Asp Thr Ala Val Tyr Tyr Cys
                85                  90                  95

Ala Arg Gly Gly Ile Val Val Pro Gly Leu Phe Glu Tyr Trp Gly Gln
            100                 105                 110

Gly Ser Leu Val Thr Val Ser Ser
        115                 120

<210>  18
<211>  6
<212>  PRT
<213>  Artificial Sequence

<220>
<223>  VH-CDR

<400>  18

Ser Ser Tyr Gly Met His
1               5

<210>  19
<211>  17
<212>  PRT
<213>  Artificial Sequence

<220>
<223>  VH-CDR

<400>  19

Val Val Trp Tyr Asp Gly Ser Asn Lys Tyr Tyr Ala Asp Ser Val Lys
1               5                   10                  15

Gly

<210>  20
<211>  13
<212>  PRT
<213>  Artificial Sequence

<220>
<223>  VH-CDR

<400>  20

Ala Arg Gly Gly Ile Val Val Pro Gly Leu Phe Glu Tyr
1               5                   10

<210>  21

<211>  107
<212>  PRT
<213>  Artificial Sequence

<220>
<223>  31C12F3_VL

<400>  21

Asp Ile Gln Met Thr Gln Ser Pro Ser Ser Leu Ser Ala Ser Val Gly
1               5                   10                  15

Asp Arg Val Ala Ile Thr Cys Arg Thr Ser Gln Ser Ile Ser Asn Leu
            20                  25                  30

Leu Asn Trp Tyr Gln Gln Lys Pro Gly Lys Ala Pro Glu Leu Leu Ile
        35                  40                  45

Tyr Ala Pro Ser Ser Phe Gln Ser Gly Val Pro Ser Arg Phe Arg Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Phe
                85                  90                  95

Thr Phe Gly Pro Gly Thr Lys Val Asp Ile Lys
            100                 105


<210>  22
<211>  11
<212>  PRT
<213>  Artificial Sequence

<220>
<223>  VL-CDR

<400>  22

Thr Ser Gln Ser Ile Ser Asn Leu Leu Asn Trp
1               5                   10


<210>  23
<211>  9
<212>  PRT
<213>  Artificial Sequence

<220>
<223>  VL-CDR

<400>  23

Ala Pro Ser Ser Phe Gln Ser Gly Val
1               5

<210>  24
<211>  9
<212>  PRT
<213>  Artificial Sequence

<220>
<223>  VL-CDR

<400>  24

Gln Gln Ser Tyr Ser Thr Pro Phe Thr
1               5


<210>  25
<211>  120
<212>  PRT
<213>  Artificial Sequence

<220>
<223>  40C4C6_VH

<400>  25

Gln Val Gln Leu Val Glu Ser Gly Gly Gly Val Val Gln Pro Gly Arg
1               5                   10                  15


Ser Leu Arg Leu Ser Cys Ala Ala Ser Gly Phe Thr Phe Ser Ser Tyr
            20                  25                  30


Gly Met His Trp Val Arg Gln Ala Pro Gly Lys Gly Leu Glu Trp Val
        35                  40                  45


Ala Val Ile Trp Tyr Asp Gly Arg Asn Lys Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Ala Ser Gly Ser Tyr Ser Tyr Tyr Phe Asp Tyr Trp Gly Gln
            100                 105                 110


Gly Thr Leu Val Thr Val Ser Ser
        115                 120


<210>  26
<211>  6
<212>  PRT
<213>  Artificial Sequence

<220>
<223>  VH-CDR

<400>  26

Ser Ser Tyr Gly Met His
1               5


<210>  27
<211>  17
<212>  PRT
<213>  Artificial Sequence

<220>
<223>  VH-CDR

<400>  27

Val Ile Trp Tyr Asp Gly Arg Asn Lys Tyr Tyr Ala Asp Ser Val Lys
1               5                   10                  15


Gly



<210>  28
<211>  13
<212>  PRT
<213>  Artificial Sequence

<220>
<223>  VH-CDR

<400>  28

Ala Arg Ala Ser Gly Ser Tyr Ser Tyr Tyr Phe Asp Tyr
1               5                   10


<210>  29
<211>  107
<212>  PRT
<213>  Artificial Sequence

<220>
<223>  40C4C6_VL

<400>  29

Asp Ile Gln Met Thr Gln Ser Pro Ser Ser Leu Ser Ala Ser Val Gly
1               5                   10                  15


Gly Arg Val Thr Ile Thr Cys Arg Ala Ser Gln Ser Ile Ser Ser Phe
            20                  25                  30


Leu Asn Trp Tyr Gln His Glu Leu Gly Lys Ala Pro Lys Leu Leu Ile
        35                  40                  45

Tyr Gly Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Thr Tyr Ser Thr Pro Phe
                85                  90                  95

Thr Phe Gly Pro Gly Thr Lys Val Asp Ile Lys
            100                 105

<210>  30
<211>  11
<212>  PRT
<213>  Artificial Sequence

<220>
<223>  VL-CDR

<400>  30

Ala Ser Gln Ser Ile Ser Ser Phe Leu Asn Trp
1               5                   10

<210>  31
<211>  9
<212>  PRT
<213>  Artificial Sequence

<220>
<223>  VL-CDR

<400>  31

Gly Ala Ser Ser Leu Gln Ser Gly Val
1               5

<210>  32
<211>  9
<212>  PRT
<213>  Artificial Sequence

<220>
<223>  VL-CDR

<400>  32

Gln Gln Thr Tyr Ser Thr Pro Phe Thr
1               5

<210>  33
<211>  121
<212>  PRT
<213>  Artificial Sequence

<220>
<223>  97B8E1_VH

<400>  33

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Ser Tyr
            20                  25                  30

Val Met His Trp Val Arg Gln Ala Pro Gly Lys Gly Leu Glu Trp Val
        35                  40                  45

Ala Val Ile Trp Ser Asp Gly Arg Asn Lys Tyr Tyr Thr Asp Ser Val
    50                  55                  60

Lys Gly Arg Phe Thr Ile Ser Arg Asp Asn Ser Lys Asn Thr Leu Tyr
65                  70                  75                  80

Leu Gln Met Ser Ser Leu Arg Ala Glu Asp Thr Ala Val Tyr Tyr Cys
                85                  90                  95

Ala Arg Ser Gly Ile Ala Leu Ala Gly Asn Ala Phe Asp Ile Trp Gly
            100                 105                 110

Gln Gly Thr Met Val Thr Val Ser Ser
        115                 120


<210>  34
<211>  6
<212>  PRT
<213>  Artificial Sequence

<220>
<223>  VH-CDR

<400>  34

Ser Ser Tyr Val Met His
1               5


<210>  35
<211>  17
<212>  PRT
<213>  Artificial Sequence

<220>
<223>  VH-CDR

<400>  35

Val Ile Trp Ser Asp Gly Arg Asn Lys Tyr Tyr Thr Asp Ser Val Lys
1               5                   10                  15

Gly

<210> 36
<211> 14
<212> PRT
<213> Artificial Sequence

<220>
<223> VH-CDR

<400> 36

Ala Arg Ser Gly Ile Ala Leu Ala Gly Asn Ala Phe Asp Ile
1               5                   10

<210> 37
<211> 107
<212> PRT
<213> Artificial Sequence

<220>
<223> 97B8E1_VL

<400> 37

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Phe
                85                  90                  95

Thr Phe Gly Pro Gly Thr Lys Val Asp Ile Lys
            100                 105

<210> 38
<211> 11
<212> PRT
<213> Artificial Sequence

<220>
<223>  VL-CDR

<400>  38

Ala Ser Gln Ser Ile Ser Ser Tyr Leu Asn Trp
1               5                   10


<210>  39
<211>  9
<212>  PRT
<213>  Artificial Sequence

<220>
<223>  VL-CDR

<400>  39

Ala Ala Ser Ser Leu Gln Ser Gly Val
1               5


<210>  40
<211>  9
<212>  PRT
<213>  Artificial Sequence

<220>
<223>  VL-CDR

<400>  40

Gln Gln Ser Tyr Ser Thr Pro Phe Thr
1               5


<210>  41
<211>  354
<212>  DNA
<213>  Artificial Sequence

<220>
<223>  97A8D1_VH

<400>  41
caggtgcagc tggtggagtc tgggggaggc gtggtccagc ctgggaggtc cctgagactc      60

tcctgtgtag cgtctggatt caccttcaat aactatggca tgaactgggt ccgccaggct     120

ccaggcaagg ggctggagtg ggtggcagtt atttggtatg gtggacgtaa taaattctat     180

gcagactccg tgaagggccg attcaccatt tccagagaca attccaagaa cacgctgtat     240

ctggaaatga acagcctgag agccgaggac acggctgtgt attactgtgc gagagctgac     300

tggggagggt ggttcgaccc ctggggccag ggaaccctgg tcaccgtctc ctca           354


<210>  42
<211>  324
<212>  DNA
<213>  Artificial Sequence

<220>
<223>  97A8D1_VL

<400>  42
gaaattgtgt tgacgcagtc tccaggcacc ctgtctttgt ctccagggga aagagccacc      60

ctctcctgca gggccagtca gagtgttagc agcacctact tagcctggta ccagcagaaa     120

cctggccagg ctcccaggct cctcatccat ggtgcattca gcagggccac tggcatccca     180

gacaggttca gtggcagtgg gtctgggcca gacttcactc tcaccatcag cagactggag     240

cctgaagatt ttgcagttta ttactgtcag cagtatggta cctcaccatt cactttcggc     300

cctgggacca aagtggatat caaa                                            324


<210>  43
<211>  360
<212>  DNA
<213>  Artificial Sequence

<220>
<223>  92C8B6_VH

<400>  43
caggtgcagc tggtggagtc tgggggaggc gtggtccagc ctgggaggtc cctgagactc      60

tcctgtgcag cgtctggatt caccttcagt aactttggca tgcactgggt ccgccaggct     120

ccaggcaagg ggctggagtg ggtggcagtt atatggtatg atggaagtaa taaatactat     180

gcagactccg tgaagggccg attcaccatc tccagagaca attccaagaa cacgctgttt     240

ctgcaaatga acggcctgag agccgaggac acggctgtgt attactgtgc gaaagggggt     300

atattaatgg ctggtacgct ggactactgg ggccagggaa ccctggtcac cgtctcctca     360


<210>  44
<211>  321
<212>  DNA
<213>  Artificial Sequence

<220>
<223>  92C8B6_VL

<400>  44
gacatccaga tgacccagtc tccatcctcc ctgtctgcat ctgtaggaga cagagtcacc      60

atcacttgcc gggcaagtca gagcgttagc acctatttaa attggtatca gcagaaacca     120

gggaaagccc ctaagctcct gatctatgct gcatccagtt tccaaagtgg ggtcccatca     180

aggttcagtg gcagtggatc tgggacagat ttcactctca ccatcagcag tctgcaacct     240

gaagattttg caacttactt ctgtcaacag agttacagta ccccattcac tttcggccct     300

gggaccaaag tggatatcaa a                                               321


<210>  45

<211>  360
<212>  DNA
<213>  Artificial Sequence

<220>
<223>  31C12F3_VH

<400>  45
caggtgcagc tggtggagtc tgggggaggc gtggtccagc ctgggaggtc cctgatactc      60

tcctgtgcag cgtctggttt caccttcagt agctatggca tgcactgggt ccgccaggct     120

ccaggcaagg ggctggagtg ggtggcagtt gtttggtatg atggaagtaa taaatactat     180

gcagactccg tgaagggccg attcaccatc tccagagaca attccaagaa cacgctgtat     240

ctgcaaatga gcagcctgag agccgaggac acggctgtgt attactgtgc gagagggggt     300

atagtagtgc ctggcctctt tgagtactgg ggccagggat ccctggtcac cgtctcctca     360


<210>  46
<211>  321
<212>  DNA
<213>  Artificial Sequence

<220>
<223>  31C12F3_VL

<400>  46
gacatccaga tgacccagtc tccatcctcc ctgtctgcat ctgtaggaga cagagtcgcc      60

atcacttgcc ggacaagtca gagcattagt aacttgttaa attggtatca gcagaaacca     120

gggaaagccc ctgagctcct gatctatgct ccatccagtt tccaaagtgg ggtcccatca     180

aggttcaggg gcagtggatc tgggacagat ttcactctca ccatcagtag tctgcaacct     240

gaagattttg caacttacta ctgtcaacag agttacagta ccccattcac tttcggccct     300

gggaccaaag tggatatcaa a                                               321


<210>  47
<211>  360
<212>  DNA
<213>  Artificial Sequence

<220>
<223>  40C4C6_VH

<400>  47
caggtgcagt tggtggagtc tgggggaggc gtggtccagc ctgggaggtc cctgagactc      60

tcctgtgcag cgtctggatt caccttcagt agctatggca tgcactgggt ccgccaggct     120

ccaggcaagg ggctggagtg ggtggcagtt atatggtatg atggaagaaa taaatattat     180

gcagactccg tgaagggccg attcaccatc tccagagaca attccaagaa cacgctgtat     240

ctgcaaatga acagcctgag agccgaggac acggctgtat attactgtgc gagggcttcg     300

gggagttatt cgtactactt tgactactgg ggccagggaa ccctggtcac cgtctcctca     360

<210>  48
<211>  321
<212>  DNA
<213>  Artificial Sequence

<220>
<223>  40C4C6_VL

<400>  48
gacatccaga tgacccagtc tccatcctcc ctgtctgcat ctgtaggagg cagagtcacc      60

atcacttgcc gggcaagtca gagcattagc agttttttaa attggtatca acatgaacta      120

gggaaagccc ctaaactcct gatctatggt gcatccagtt tgcaaagtgg ggtcccatca      180

aggttcagtg gcagtggatc tgggacagat ttcactctca ccatcagcag tctgcaacct      240

gaagattttg caacttacta ctgtcaacag acttacagta ccccattcac tttcggccct      300

gggaccaaag tggatatcaa a                                                321


<210>  49
<211>  363
<212>  DNA
<213>  Artificial Sequence

<220>
<223>  97B8E1_VH

<400>  49
caagtgcagc tggtggagtc tgggggaggc gtggtccagc ctgggaggtc cctgagactc      60

tcctgtgcag cgtctggatt caccttcagt agttatgtca tgcactgggt ccgccaggct      120

ccaggcaagg ggctggagtg ggtggcagtt atatggtctg atggaagaaa taaatactat      180

acagactccg tgaagggccg attcaccatc tccagagaca attccaagaa cacgctgtat      240

ctgcaaatga gcagcctgag agccgaggac acggctgtgt attactgtgc gcggtcgggt      300

atagcgctgg ctggtaacgc ttttgatatc tggggccaag ggacaatggt caccgtctct      360

tca                                                                    363


<210>  50
<211>  321
<212>  DNA
<213>  Artificial Sequence

<220>
<223>  97B8E1_VL

<400>  50
gacatccaga tgacccagtc tccatcctcc ctgtctgcat ctgtaggaga cagagtcacc      60

atcacttgcc gggcaagtca gagcattagc agttatttaa attggtatca gcagaaacca      120

gggaaagccc ctaagctcct gatctatgct gcatccagtt tgcaaagtgg ggtcccatca      180

aggttcagtg gcagtggatc tgggacagat ttcactctca ccatcagcag tctgcaacct 240

gaagattttg caacttacta ctgtcaacag agttacagta ccccattcac tttcggccct 300

gggaccaaag tggatatcaa a 321